(19) Europäisches Patentamt
European Patent Office
Office européen des brevets

(11) Publication number: **0 103 077**
**B1**

(12) **EUROPEAN PATENT SPECIFICATION**

(45) Date of publication of patent specification: **18.05.88**

(21) Application number: **83105748.4**

(22) Date of filing: **11.06.83**

(51) Int. Cl.⁴: **C 07 C 103/84**, C 07 K 5/02, C 07 D 307/54, C 07 D 317/24, C 07 D 309/30, C 07 D 333/24, A 61 K 31/165, A 61 K 31/33, A 61 K 37/02

(54) **Substituted dipeptides, methods for their production, pharmaceutical compositions containing them, method for making such pharmaceutical compositions.**

(30) Priority: **17.06.82 NZ 201001**
**26.11.82 US 444761**
**11.04.83 US 483463**

(43) Date of publication of application:
**21.03.84 Bulletin 84/12**

(45) Publication of the grant of the patent:
**18.05.88 Bulletin 88/20**

(84) Designated Contracting States:
**AT BE CH DE FR GB IT LI LU NL SE**

(56) References cited:
**EP-A-0 012 401**
**EP-A-0 038 758**
**EP-A-0 050 800**
**EP-A-0 054 862**

**The file contains technical information submitted after the application was filed and not included in this specification**

(73) Proprietor: **SCHERING CORPORATION**
**2000 Galloping Hill Road**
**Kenilworth, New Jersey 07033 (US)**

(72) Inventor: **Berger, Joel G.**
**54 Park Avenue**
**Verona New Jersey 07044 (US)**

(74) Representative: **von Kreisler, Alek, Dipl.-Chem et al**
**Patentanwälte Dr.-Ing. Schönwald Dr.-Ing. Eishold; Dr. Fues Dipl.-Chem. von Kreisler; Dipl.Chem. Keller Dipl.-Ing. Selting; Dr. Werner**
**Deichmannhaus am Hauptbahnhof**
**D-5000 Köln 1 (DE)**

**EP 0 103 077 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Enkephalin is a natural opiate receptor agonist and is believed to be a mixture of two pentapeptides:

H—Tyr—Gly—Gly-Phe—Met—OH (methionine-enkephalin), and
H—Tyr—Gly—Gly—Phe—Leu—OH (leucine-enkephalin).

Hereinafter, the name enkephalin is used generically to embrace all such compounds.

It has been reported by Beluzzi et al., Nature, 260, 625 (1976), that when enkephalin is injected into the brain ventricle of rats, a profound analgesia is obtained. It is also known in the art that enkephalin is acted upon by a group of enzymes known generically as enkephalinases, which are also naturally occurring and is inactivated thereby.

European Patent Application No. 79105015.6, publication No. 12401 discloses certain dipeptide derivatives which are described as possessing antihypertensive effects.

European Patent Application No. 81110337.3 publication No. 54862 discloses certain dipeptide compounds which possess an enkephalinase inhibitory activity.

The present invention comprises compounds having the formula I

$$R_1C^*(HCOR_2)—NH—C^*(HR_3)—CONH(CH_2)p\text{-}C^*(R_4R_5)—COR_6$$

or a mixture of enantiomers or diasterioisomers containing the same, and the pharmaceutically acceptable salts thereof wherein:

$R_1$ is alkyl having from 1 to 6 carbon atoms, adamantylmethyl, cycloalkylmethyl having from 4 to 8 carbon atoms or $A—X_m—C_nH_{2n}—$ wherein X is oxygen or sulfur, A is phenyl which may be substituted with the group Y, wherein Y is halogen, hydroxy, trifluoromethyl, alkoxy having from 1 to 6 carbon atoms, alkyl having from 1 to 6 carbon atoms, 2- or 3-furanyl, 2- or 3-thienyl, or phenyl [which may substituted with halogen, hydroxy, trifluoromethyl, alkoxy having from 1 to 6 carbon atoms or alkyl having from 1 to 6 carbon atoms] or may also be benzyl [the phenyl ring of which may be substituted with the group Y, as defined above], 1- or 2-naphthyl, 2- or 3-furanyl or 2- or 3-thienyl; m is 0 or 1 and n is 0, 1, 2, 3, or 4;

$R_2$ and $R_6$ may be the same or different and are hydroxy, alkoxy having from 1 to 8 carbon atoms, $B—X_m—C_nH_{2n}—O—$ wherein B is phenyl [which may be substituted with the group Y, as defined herein] or 1- or 2-naphthyl, X, m, and n are as defined herein provided that when n=0, m=0, $—OCH_2OCO$-alkyl in which the alkyl group has 1 to 6 carbon atoms, $—OCH_2CO$-phenyl [the phenyl ring of which may be substituted with the group Y, as defined above], 1-glyceryl,

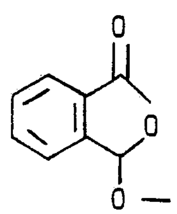

or

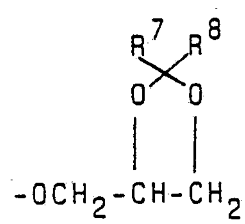

wherein $R_7$ is hydrogen, alkyl having from 1 to 6 carbon atoms, or phenyl which may be substituted with the group Y, as defined above, and $R_8$ is hydrogen or alkyl having from 1 to 6 carbon atoms;

$R_2$ may also be $—NR_7R_8$ wherein $R_7$ and $R_8$ are as defined above;

$R_3$ is alkyl having from 1 to 6 carbon atoms, cycloalkylmethyl having from 4 to 8 carbon atoms, 2- or 3-thienylmethyl, 2- or 3-furanylmethyl, 1- or 2-naphthylmethyl, or benzyl the phenyl ring of which may be substituted with the group Y, as defined above;

$R_4$ is $D—C_nH_{2n}—O_m—$ wherein D is hydrogen, alkyl having from 1 to 4 carbon atoms or phenyl which may be substituted with the group Z, wherein Z is halogen, hydroxy, trifluoromethyl, alkoxy having from 1 to 6 carbon atoms, or alkyl having from 1 to 6 carbon atoms;

m and n are as defined above;

$R_5$ is hydrogen or alkyl having from 1 to 6 carbon atoms; and

p is 0, 1 or 2;

subject to the following provisos

(i) that when $R_4$ and $R_5$ are both hydrogen, and p is 1 or 2 then;

a) $R_1$ is chosen from adamantyl methyl, 4-phenylphenylethyl, $A—X_m—C_nH_{2n}—$ wherein m is 1, X is as defined above and *n* is 1,2,3 or 4, 1- or 2-napthyl, 2- or 3-furanyl and 2- or 3-thienyl; and/or

b) $R_2$ and/or $R_6$ are chosen from $B—Xm—CnH_{2n}—O—$ wherein B is as defined above, m is one, X is as defined above and n is 1,2,3 or 4, $—OCH_2OCO$-alkyl in which the alkyl group has 1 to 6 carbon atoms, $—OCH_2CO$-phenyl (in which the phenyl group may be substituted by the group Y, as defined herein), 1-glyceryl,

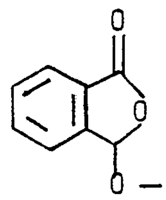 or 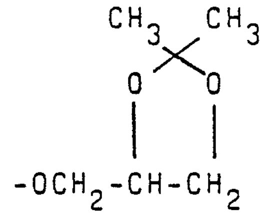

and $R_2$ may also be $—NR_7R_8$ wherein $R_7$ and $R_8$ are as defined herein; and/or

c) $R_3$ is 2- or 3-furanylmethyl or 4-phenyl-phenylethyl;

(ii) when P is zero then

$R_1$ is benzyl or benzylthiomethyl;

$R_3$ is benzyl or maybe leucyl when $R_1$ is benzyl;

$R_4$ is benzyl when $R_1$ is benzyl, and is methyl when $R_1$ is benzylthiomethyl;

$R_5$ is hydrogen.

In formula I, the asterisks denote those carbon atoms which may be asymmetric (chiral) centres. The invention contemplates all isomers at these centres both in pure form and in admixture.

One group of compounds of formula I of interest are those in which $R_4$ represents a group $D—Cn H_{2n}—O_m—$ as defined above in which the sum of n and m is at least one, that is $R_4$ does not represent hydrogen. Examples of such $R_4$ groups are hydroxy, methoxy, methyl and benzyl with preferred values being methyl and benzyl. Preferred values for the other groups represented in formula I when $R_4$ has such value are as follows:

$R_1$ is benzyl optionally *para* substituted by chlorine, methoxy, methyl or phenyl, 2-phenylethyl or 1- or 2-naphthylmethyl and is most preferably benzyl or p-phenylbenzyl,

$R_2$ and $R_6$ are the same or different and are hydroxy, methoxy, ethoxy, benzyloxy, 2-phenoxyethoxy, 1-glyceryl,

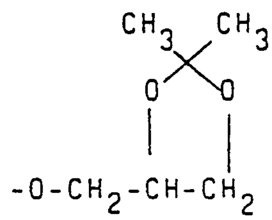, 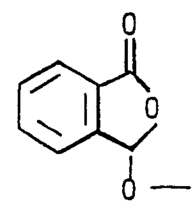,

or (2,2-dimethyl-1-oxopropoxy)methoxy, and are most preferably chosen from hydroxy, 2-phenoxyethoxy, 1-glyceryl,

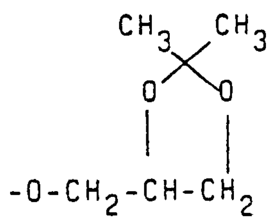, 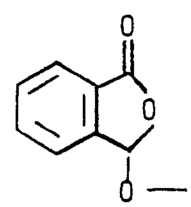,

and (2,2-dimethyl-1-oxypropoxy)methoxy,

$R_3$ is benzyl, *p*-methylbenzyl, *p*-phenylbenzyl, 1-naphthylmethyl or 3-thienylmethyl and is most preferably benzyl or *p*-phenylbenzyl, and

$R_5$ is hydrogen.

Another group of compounds of interest are those in which one of $R_2$ and $R_6$ is essentially chosen from 2-phenoxyethoxy, 1-glyceryl,

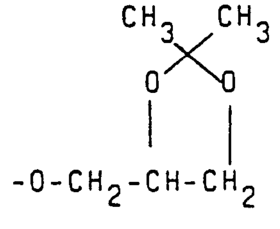, 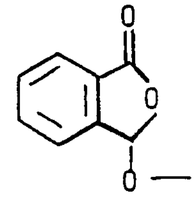,

or (2,2-dimethyl-1-oxopropoxy)methoxy and the other is chosen from the foregoing groups or is hydroxy,

methoxy, ethoxy or benzyloxy: when $R_2$ and $R_6$ have such values, the preferred values for $R_1$ $R_3$ and $R_5$ are as set forth in the preceding paragraph, and preferred values for $R_4$ are hydrogen, methyl and benzyl.

p is preferably one.

As used herein, unless stated otherwise, the terms alkyl and alkoxy denote such groups having straight or branched carbon chains of from 1 to 6 carbon atoms. The term pivaloyloxymethyl is the trivial or common name for the (2,2-dimethyl-1-oxopropoxy)methyl group.

Halogen includes fluorine, chlorine, bromine and iodine.

Certain of the compounds having structural formula I form salts with pharmaceutically acceptable acids. Hydrochloric, sulfuric, acetic, maleic, fumaric and the like may be utilized.

Compounds having structural formula I wherein $R_2$ and/or $R_6$ are hydroxy form salts with pharmaceutically acceptable bases. Sodium, potassium and calcium hydroxide as well as sodium and potassium carbonate are examples of suitable bases for this purpose. In addition, salts formed with pharmaceutically acceptable amines such as, for example, ammonia, N-methylglucamine, benzylamine and morpholine are also contemplated.

Specific compounds having structural formula I within the scope of invention are those having the names:

1. N-[N-L-1-(2,2-dimethyl-1-oxopropoxy)methoxy]carbonyl]-2-phenylethyl]-L-phenylalanyl]-β-alanine, (2,2-dimethyl-1-oxopropoxy)methyl ester;
2. N-[N-L-1-[phenylmethoxy]carbonyl]-2-phenylethyl]-L-phenylalanyl]-β-alanine, (2,2-dimethyl-1-oxopropoxy)methyl ester;
3. N-[N-L-1-carboxy-2-(4-phenyl)phenylethyl]-L-phenylalanyl]-β-alanine;
4. N-[N-L-[1-(2,2-dimethyl-1-oxopropoxy)methoxy]carbonyl]-2-(4-phenyl)phenylethyl]-L-phenylalanyl]-β-alanine, (2,2-dimethyl-1-oxopropoxy)methyl ester;
5. N-[N-L-1-carboxy-2-phenylethyl]-L-(4-phenyl)phenylalanyl]-β-alanine;
6. N-[N-[L-[1-[(2,2-dimethyl-1-oxopropoxy)methoxy]carbonyl]-2-(4-phenyl)phenylethyl]-L-(4-phenyl)phenylalanyl-β-alanine, (2,2-dimethyl-1-oxopropoxy)methyl ester;
7. N-[N-[L-[1-[(2,2-dimethyl-1-oxopropoxy)methoxy]carbonyl]-2-phenylethyl]-L-(4-phenyl)phenylalanyl]-β-alanine, (2,2-dimethyl-1-oxopropoxy)methyl ester;
8. N-[N-L-1-carboxy-2-phenylethyl]-L-phenylalanyl]-L-(α-methyl)-β-alanine;
9. N-[N-[L-[1-[(2,2-dimethyl-1-oxopropoxy)methoxy]carbonyl]-2-phenylethyl]-L-phenylalanyl]-L-(α-methyl)-β-alanine, (2,2-dimethyl-1-oxopropoxy)methyl ester;
10. N-[N-[(L-1-carboxy-2-phenylethyl)]-L-phenylalanyl]-β-alanine, 2-phenoxyethyl ester;
11. N-[N-[(D-1-carboxy-2-phenylethyl)]-L-leucyl]-L-phenylalanine;
12. N-[N-[(L-1-carboxy-2-phenylethyl)]-L-phenylalanyl]-L-phenylalanine;
13. N-[N-[(L-1-carboxy-3-phenylpropyl)]-L-phenylalanyl]-β-alanine;
14. N-[N-[L-[1-[(2,2-dimethyl-1-oxopropoxy)methoxy]carbonyl]-2-phenylethyl]-L-phenylalanyl]-β-alanine;
15. N-[N-[L-[1-[(2-phenoxy)ethoxy]carbonyl]-2-phenylethyl]-L-phenylalanyl]-β-alanine;
16. N-[N-[L-[1-[(1-oxo-3-isobenzofuranyloxy)]carbonyl]-2-phenylethyl]-L-phenylalanyl-β-alanine;
17. N-[N-[L-[1-[(2,3-dihydroxy)-1-propoxy]carbonyl]-2-phenylethyl]-L-phenylalanyl]-β-alanine;
18. N-[N-[L-[1-[(2,2-dimethyl-1,3-dioxolan-4-yl)methoxy]carbonyl]-2-phenylethyl]-L-phenylalanyl]-β-alanine;
19. N-[N-[L-[1-[(2,2-dimethyl-1-oxopropoxy)methoxy]carbonyl]-2-(4-phenyl)phenylethyl]-L-phenylalanyl]-β-alanine;
20. N-[N-[L-[1-[(2-phenoxy)ethoxy]carbonyl]-2-(4-phenyl)phenylethyl]-L-phenylalanyl]-β-alanine;
21. N-[N-[L-[1-[(1-oxo-3-isobenzofuranyloxy)]carbonyl]-2-(4-phenyl)phenylethyl]-L-phenylalanyl]-β-alanine;
22. N-[N-[L-[1-[(2,3-dihydroxy)-1-propoxy]carbonyl]-2-(4-phenyl)phenylethyl]-L-phenylalanyl]-β-alanine;
23. N-[N-[L-[1-[(2,2-dimethyl-1,3-dioxolan-4-yl)methoxy]carbonyl]-2-(4-phenyl)phenylethyl]-L-phenylalanyl]-β-alanine;
24. N-[N-[L-[1-[(2,2-dimethyl-1-oxopropoxy)methoxy]carbonyl]-2-phenylethyl]-L-(4-phenyl)phenylalanyl]-β-alanine;
25. N-[N-[L-[1-[(2-phenyl)ethoxy]carbonyl]-2-phenylethyl]-L-(4-phenyl)phenylalanyl]-β-alanine;
26. N-[N-[L-[1-[(1-oxo-3-isobenzofuranyloxy)]carbonyl]-2-phenylethyl]-L-(4-phenyl)phenylalanyl]-β-alanine;

27 N-[N-[L-[1-[(2,3-dihydroxy)-1-propoxy]carbonyl]-2-phenylethyl]-L-(4-phenyl)phenylalanyl]-β-alanine;

28. N-[N-[L-[1-[(2,2-dimethyl-1,3-dioxolan-4-yl)methoxy]carbonyl]-2-phenylethyl]-L-(4-phenyl)phenylalanyl]-β-alanine;
29. N-[N-[L-[1-[(2,2-dimethyl-1-oxopropoxy)methoxy]carbonyl]-2-(4-phenyl)phenylethyl]-L-(4-phenyl)phenylalanyl]-β-alanine;
30. N-[N-[L-[1-[(2-phenoxy)ethoxy]carbonyl]-2-(4-phenyl)phenylethyl]-L-(4-phenyl)phenylalanyl]-β-alanine;
31. N-[N-[L-[1-[(1-oxo-3-isobenzofuranyloxy)]carbonyl]-2-(4-phenyl)phenylethyl]-L-(4-phenyl)phenylalanyl]-β-alanine;

32. N-[N-[L-[1-[(2,3-dihydroxy)-1-propoxy]carbonyl]-2-(4-phenyl)phenylethyl]-L-(4-phenyl)phenylalanyl]-β-alanine;

33. N-[N-[L-[1-[(2,2-dimethyl-1,3-dioxolan--4yl)methoxy]carbonyl]-2-(4-phenyl)phenylethyl]-L-(4-phenyl)phenylalanyl]-β-alanine;

34. N-[N-[L-[1-[(2,2-dimethyl-1-oxopropoxy)methoxy]carbonyl]-2-phenylethyl]-L-phenylalanyl]-D,L-α-methyl-β-alanine;

35. N-[N-[L-[1-[(2-phenoxy)ethoxy]carbonyl]-2-phenylethyl]-L-phenylalanyl]-D,L-α-methyl-β-alanine;

36. N-[N-[L-[1-[(1-oxo-3-isobenzofuranyloxy)]carbonyl]-2-phenylethyl]-L-phenylalanyl]-D,L-α-methyl-β-alanine;

37. N-[N-[L-[1-[(2,3-dihydroxy)-1-propoxy]carbonyl]-2-phenylethyl]-L-phenylalanyl]-D,L-α-methyl-β-alanine;

38. N-[N-[L-[1-[(2,2-dimethyl-1,3-dioxolan-4-yl)methoxy]carbonyl]-2-phenylethyl]-L-phenylalanyl]-D,L-α-methyl-β-alanine;

39. N-[N-[L-[1-[(2,2-dimethyl-1-oxopropoxy)methoxy]carbonyl]-2-(4-phenyl)phenylethyl]-L-phenylalanyl]-D,L-α-methyl-β-alanine;

40. N-[N-[L-[1-[(2-phenoxy)ethoxy]carbonyl]-2-(4-phenyl)phenylethyl]-L-phenylalanyl]-D,L-α-methyl-β-alanine;

41. N-[N-[L-[1-[(1-oxo-3-isobenzofuranyloxy)]carbonyl]-2-(4-phenyl)phenylethyl]-L-phenylalanyl]-D,L-α-methyl-β-alanine;

42. N-[N-[L-[1-[(2,3-dihydroxy)-1-propoxy]carbonyl]-2-(4-phenyl)phenylethyl]-L-phenylalanyl]-D,L-α-methyl-β-alanine;

43. N-[N-[L-[1-[(2,2-dimethyl-1,3-dioxolan-4-yl)methoxy]carbonyl]-2-(4-phenyl)phenylethyl]-L-phenylalanyl]-D,L-α-methyl-β-alanine;

44. N-[N-[L-[1-[(2,2-dimethyl-1-oxopropoxy)methoxy]carbonyl]-2-phenylethyl]-L-(4-phenyl)phenylalanyl]-D,L-α-methyl-β-alanine;

45. N-[N-[L-[1-[(2-phenoxy)ethoxy]carbonyl]-2-phenylethyl]-L-(4-phenyl)phenylalanyl]-D,L-α-methyl-β-alanine;

46. N-[N-[L-[1-[(1-oxo-3-isobenzofuranyloxy)]carbonyl]-2-phenylethyl]-L-(4-phenyl)phenylalanyl]-D,L-α-methyl-β-alanine;

47. N-[N-[L-[1-[(2,3-dihydroxy)-1-propoxy]carbonyl]-2-phenylethyl]-L-(4-phenyl)phenylalanyl]-D,L-α-methyl-β-alanine;

48. N-[N-[L-[1-[(2,2-dimethyl-1,3-dioxolan-4-yl)methoxy]-carbonyl]-2-phenylethyl]-L-(4-phenyl]phenylalanyl]-D,L-α-methyl-β-alanine;

49. N-[N-[L-]1-](2,2-dimethyl-1-oxopropoxy)methoxy]carbonyl]-2-(4-phenyl)phenylethyl]-L-(4-phenyl)phenylalanyl]-D,L-α-methyl-β-alanine;

50. N-[N-[L-[1-[(2-phenoxy)ethoxy]carbonyl]-2-(4-phenyl)-phenylethyl]-L-(4-phenyl)phenylalanyl]-D,L-α-methyl-β-alanine;

51. N-[N-[L-[(1-oxo-3-isobenzofuranyloxy)]carbonyl]-2-(4-phenyl)phenylethyl]-L-(4-phenyl)phenylalanyl]-D,L-α-methyl-β-alanine;

52. N-[N-[L-[1-[(2,3-dihydroxy)-1-propoxy]carbonyl]-2-(4-phenyl)phenylethyl]-L-(4-phenyl)phenylalanyl]-D,L-α-methyl-β-alanine;

53. N-[N-[L-[1-[(2,2-dimethyl-1,3-dioxolan-4-yl)methoxy]-carbonyl]-2-(4-phenyl)phenylethyl]-L-(4-phenyl)phenylalanyl]-D,L-α-methyl-β-alanine;

54. N-[N-[L-[1-[(2,2-dimethyl-1-oxopropoxy)methoxy]carbonyl]-2-phenylethyl]-L-2-thienylalanyl]-β-alanine;

55. N-[N-[L-[1-[(2,2-dimethyl-1-oxopropoxy)methoxy]carbonyl]-2-phenylethyl]-L-3-thienylalanyl]-β-alanine;

56. N-[N-[L-[1-[(2,2-dimethyl-1-oxopropoxy)methoxy]carbonyl]-2-phenylethyl]-L-2-furoalanyl]-β-alanine;

57. N-[N-[L-[1-[(2,2-dimethyl-1-oxopropoxy)methoxy]carbonyl]-2-phenylethyl]-L-phenylalanyl]-α-methylalanine;

58. N-[N-[L-[1-[(2,2-dimethyl-1-oxopropoxy)methoxy]carbonyl]-2-phenylethyl]-L-phenylalanyl]-L-α-hydroxy-β-alanine;

59. N-[N-[L-[1-[(2,2-dimethyl-1-oxopropoxy)methoxy]carbonyl]-2-phenylethyl]-L-phenylalanyl]-D-α-hydroxy-β-alanine;

60. N-[N-[L-[1-[(2,2-dimethyl-1-oxopropoxy)methoxy]carbonyl]-2-phenylethyl]-L-phenylalanyl]-L-α-methoxy-β-alanine;

61. N-[N-[L-[1-[(2,2-dimethyl-1-oxopropoxy)methoxy]carbonyl]-2-phenylethyl]-L-phenylalanyl]-D-α-methoxy-β-alanine;

62. N-[N-[L-[(2,2-dimethyl-1-oxopropoxy)methoxy]carbony]-2-phenylethyl]-L-phenylalanyl]-D,L-α-methyl-β-alanine benzyl ester; and

63. N-[N-[L-[1-[(2,2-dimethyl-1-oxopropoxy)methoxy]carbonyl]-2-(4-methoxy)phenylethyl]-L-phenylalanyl-β-alanine.

Also within the scope of the invention are the pharmaceutically acceptable salts of the above listed specific compounds.

The compounds having structural formula I may be utilized to exert their analgesic effect in the many dosage forms known to the art, such as tablets, capsules or elixirs for oral administration or in sterile solutions or suspensions for parenteral administration. The foregoing pharmaceutical dosage forms are advantageously prepared using, in addition to a compound of this invention, pharmaceutically acceptable and compatible excipients, binders, preservatives, stabilizers, flavours and the like. In each of the dosage forms the active compound will be administered in a dosage in the range of from about 1 to 10 mg/kg. The doses are to be administered at intervals of from 3 to 8 hours. However, the quantity and frequency of dosage will depend upon such factors as the severity of the pain, the general physical condition of the patient, including the age and weight of the patient and other factors which a person skilled in the art will recognize.

The compounds of this invention may be prepared by using reactions and reagents well known in the polypeptide art.

Broadly speaking the compounds of formula I are prepared by an appropriate one of the following methods (in which in the following formula p, $R_1$, $R_2$, $R_3$, $R_4$, $R_5$ and $R_6$ are as defined above including suitable protection of any reactive groups);

a) reducing a compound of formula XX at the C=N double bond

$$R_1\text{—}\underset{\displaystyle COR_2}{\underset{|}{C}}\text{=N—}\overset{\displaystyle R_3}{\overset{|}{C}H}\text{—CONH}(CH_2)p\text{-}CR_4R_5COR_6 \rightarrow I;$$

XX

b) condensing a keto acid or ester of formula II with an amino acid of formula III in a reactive medium containing a reducing agent

$$R_1\text{—C(O)}COR_2 + H_2N\overset{\displaystyle R_3}{\overset{|}{C}}HCONH(CH_2)p\ CR_4R_5COR_6 \rightarrow I;$$

II III

c) coupling an amino acid of formula VIII with an amino acid of formula V

$$R_1\text{—}\underset{\displaystyle COR_2}{\underset{|}{C}}\ NH\overset{\displaystyle R_3}{\overset{|}{C}H}\text{—}CO_2H + H_2N(CH_2)p\ CR_4R_5\ COR_6 \rightarrow I$$

VII V

d) reducing a compound of the formula XXI at the C=N double bond

$$R_1\text{—}\underset{\displaystyle COR_2}{\underset{|}{C}H}\text{—N=}\overset{\displaystyle R_3}{\overset{|}{C}}\text{—CONH}(CH_2)pCR_4R_5COR_6 \rightarrow I$$

XXI

e) condensing a keto acid or ester of formula X with an amino acid in a reactive medium containing a reducing agent

$$O\text{=}\overset{\displaystyle R_3}{\overset{|}{C}}\text{—CONH}(CH_2)pCR_4R_5COR_6 + R_1\text{—}\underset{\displaystyle COR_2}{\underset{|}{C}HNH_2} \rightarrow I$$

X

in a reactive medium containing a reducing agent;

f) alkylating an amine of formula III with a compound of formula XXII

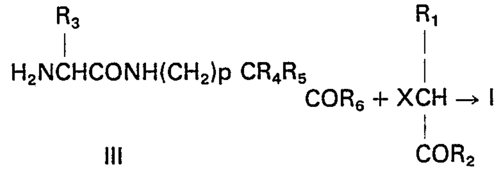

III XXII

in which X is a halogen atom; followed by removal of any protecting groups if necessary to obtain the desired compound of formula I, and thereafter, if desired converting the compound of formula I into another compound of formula I and/or forming a salt thereof, and if desired isolating a preferred isomer.

The following overall procedures may be used to prepare the compounds of this invention from readily available or easily prepared starting materials:

Procedure 1

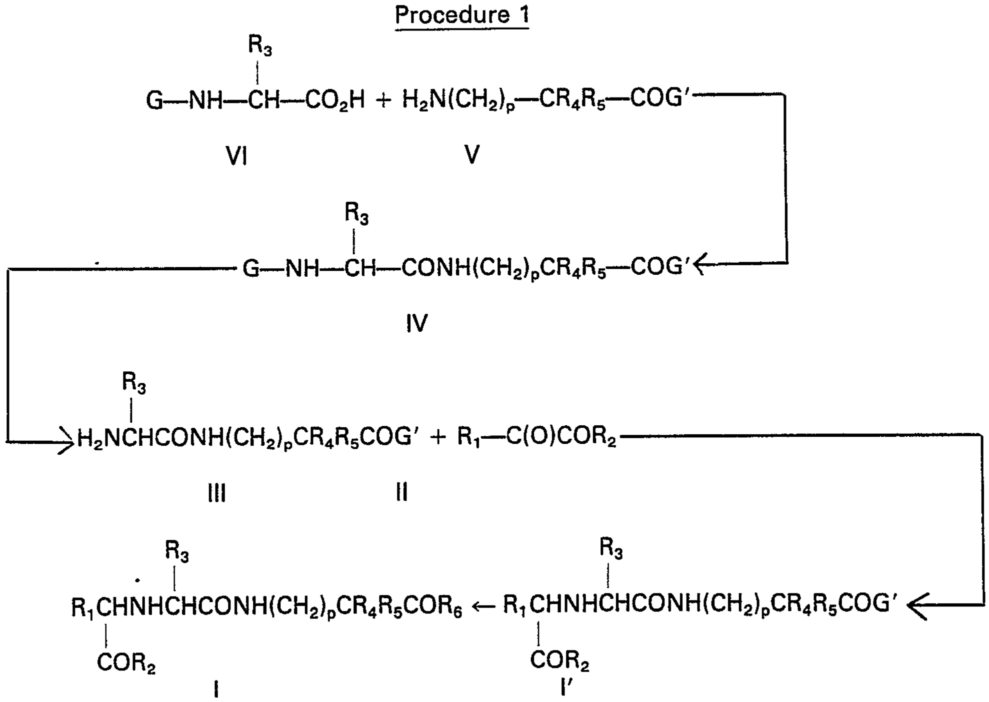

VI V IV III II I I'

In the foregoing reaction sequence, the amino function of Compound VI is protected by an amino protecting group commonly used in the art (G) such as benzyloxycarbonyl, *t*-butyloxycarbonyl or the like. Compound VI is condensed with an aminoester derivative V wherein G' is benzyloxy, *t*-butyloxy, lower-alkoxy or the like. Condensing agents such as dicyclohexyl carbodiimide, or diphenylphosphoryl azide may be employed. Also, activating agents such as 1-hydroxybenzotriazole may be employed in the reaction.

The resulting dipeptide IV is deprotected at the amine terminus by treatment with acids or by hydrogenation using for example, hydrogen and a metal catalyst. The resulting product (III), is then condensed (according to method (b) as defined above) with a ketoacid or ketoester (II) in a suitable solvent such as water or acetonitrile at a substantially neutral pH in the presence of a reducing agent such as sodium cyanoborohydride or other equivalently functioning reducing agent. Alternatively, the Schiff base resulting from the initial condensation of II and III may be reduced (according to method (a) as defined above) e.g. by catalytic reduction to give I using hydrogen at a pressure of 1—4 atmospheres. The catalytic reduction may be effected using Raney nickel catalyst or 10% palladium on carbon or the like. The compounds of formula I having a terminal carboxyl group may be prepared from a corresponding ester by hydrolysis or hydrogenolysis.

In the foregoing reaction sequence, substituents $R_1$, $R_2$, $R_3$, $R_4$, $R_5$ and $R_6$; and p are as previously defined, G and G' are suitable protecting groups, e.g. benzyloxy carbonyl or t-butyloxy carbonyl.

Procedure 2

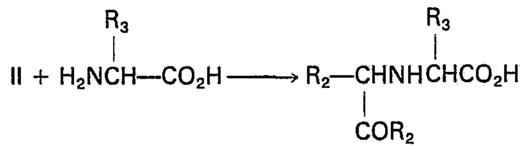

VII VIII

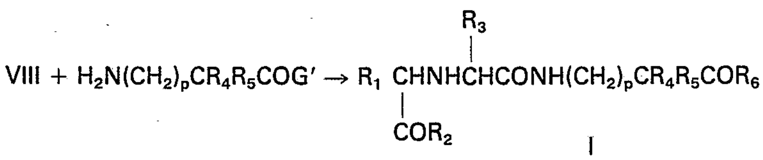

I

Procedure 2 is commenced by condensing an $R_3$ substituted amino acid with a ketoester II by procedures substantially as described in Procedure 1. The resulting intermediate (VIII) is then coupled (according to method (c) as defined above) with an $R_4$, $R_5$ substituted amino acid wherein the carboxy group is derivatized by a lower alkoxy, or a dialkylamino group, or an equivalently acting carboxy protective group G′ to form compounds which after removal of protecting groups which may be present will produce products of this invention wherein substituents $R_1$, $R_2$, $R_3$, $R_4$, $R_5$ and $R_6$; and p are as defined above.

Procedure 3

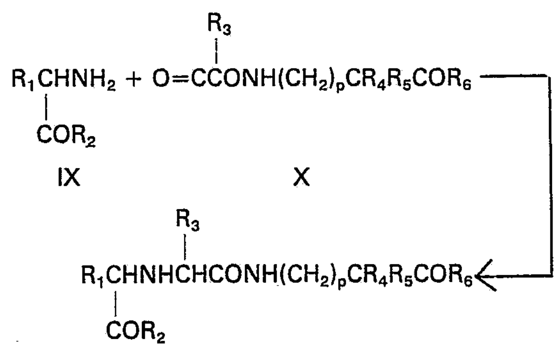

IX X

I

An amino acid or ester (IX) wherein $R_1$ and $R_2$ are defined as above is condensed with a carbonyl compound (X) under conditions described in Procedures 1 and 2 to prepare I (according to methods (d) or (e) as defined above). Substituents $R_3$, $R_4$, $R_5$ and $R_6$; and p are as previously described.

Procedure 4

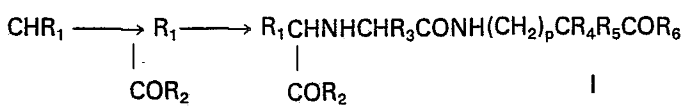

I

Intermediate III, whose preparation is shown in Procedure 1, is reacted with a substituted halo ester under conventional alkylating conditions, preferably in the presence of a base (a tertiary amine or an inorganic hydroxide, carbonate or bicarbonate). The reaction is usually carried out in water or in an organic solvent, such as *N,N*-dimethylformamide or acetonitrile. Substituents $R_1$, $R_2$, $R_3$, $R_4$, $R_5$ and $R_6$; and p are as previously described.

In the foregoing reaction sequences wherein water is generated by the reactants (e.g. the condensation of compounds II and III in Method 1), the reaction may be effected by azeotropic distillation of the formed water with suitable high boiling solvents such a toluene or xylene. Alternatively, the reactions may be effected in the presence of dehydrating agents such as molecular sieves or the like.

Various of the intermediates for use in the methods and procedures described above can be obtained by following directly or analogously procedures described in European Patent Application publication No. 54862.

Further a number of the intermediates for preparing the compounds of this invention are commercially available or they may be readily prepared by art recognized methods. Intermediates for preparing a

substantial number of the compounds of this invention are described or the preparation thereof is embodied in publications and treatises relating to peptide chemistry such as, J.H. Jones, in "Comprehensive Organic Chemistry", Vol. 2, D. Barton and W.D. Ollis, Editors, Pergamon Press, 1979 pp 819—823 and references 2, and 29—31 cited therein. The relevant portions of this publication are incorporated by reference herein.

The following examples illustrate the preparation of the compounds of the invention.

Example 1

N-[N-[L-1-carboxy-2-(4-phenyl)phenylethyl]-L-phenylalanyl]-β-alanine benzyl ester

A. 4-Phenylphenylpyruvic Acid

40 g of Potassium *t*-butoxide was added to 150 ml of diethyl oxalate in small portions with stirring. After the initial exothermic reaction subsided, the reaction mixture was heated on a steam bath under nitrogen in order to dissolve solids. After cooling down to room temperature, 79 g of 4-biphenyl acetic acid methyl ester was added in one portion. The mixture was stirred at 60—70°C., for 2 hours while low boiling material was removed under vacuum. On cooling to room temperature, the viscous residue was stirred with 200 ml of ether and 350 ml of water with cooling. The ether layer was separated and extracted once with 100 ml of water. The aqueous layers were combined, extracted once with ether, made acidic with concentrated HCl (cooling) and extracted with 2 × 300 ml of ether. Some solids did not dissolve in the ether layer and were filtered. The ether layer was then evaporated to dryness and the semi-solid residue was combined with the preceeding solids. A mixture of 160 ml of conc. HCl and 350 ml of acetic acid was added to the solids/semi-solid residue mixture, and the resulting mixture was heated under reflux for 2 1/2 hours. On cooling down to 50°C., a solid precipitated, which was filtered and washed with 150 ml of water. The wet solid was stirred with 150 ml of acetonitrile for five minutes, then filtered and dried under high vacuum at room temperature for 3 hours. 42.2 g of the title compound m.p. 215—218°C., were obtained.

B. N-[N-[N-tert.-butyloxycarbonyl]-L-phenylalanyl]-β-alanine benzyl ester

To a stirred mixture of 0.023 mole each of di-*t*-butyldicarbonate, L-phenylalanine, β-alanine benzyl ester p-toluenesulfonate, hydroxybenzotriazole, N-dimethylaminopropyl-N'-ethylcarbodimide hydrochloride in 75 ml of dry *N,N*-dimethylformamide in an ice bath was added 5 ml of N-ethyl-morpholine and the mixture stirred at room temperature for 3 hours, then poured into 600 ml of ice water and extracted with 3 × 150 ml of ether. The ether layers were combined and extracted once with 150 ml of 0.3N HCl, then twice with 300 ml of water, and then dried over $Na_2SO_4$, filtered and evaporated down to dryness at 28°C *in vacuo*. 9.0 g of gummy solid residue were obtained.

C. N-(L-Phenylalanyl)-β-alanine benzyl ester hydrochloride

4.0 g of the material from Example 1B in 25 ml of ethyl acetate at 0°C was stirred with gaseous HCl for 10 min. Stir at 0°C for 1 1/2 hours then at 10°C for 30 min. A stream of $N_2$ was passed into the solvent to expel excess HCl. The solution was poured into 200 ml of ether with vigorous stirring, and the precipitated solid filtered, and then dried at room temperature under high vacuum for 2 hours to give 3.35 g of product.

D. N-[N-[L-1-Carboxy-2-(4-phenyl)phenylethyl]-L-phenylalanyl]-β-alanine benzyl ester

A mixture of 9.10 g of N-(L-phenylalanyl)-β-alanine benzyl ester hydrochloride and 8.40 g of the compound of Example 1A in 500 ml of a mixture of tetrahydrofuranether (9:1) was treated with triethylamine to pH 6.6 and stirred at room temperature for 1 1/2 hours. A solution of 2.0 g of sodium cyanoborohydride in a mixture of 100 ml of tetrahydrofuranethanol was added dropwise over a period of 2 hours with stirring. Stirring was continued at room temperature overnight. The reaction mixture was concentrated to 75 ml at 40°C *in vacuo*. The residue was stirred with 400 ml each of 0.5N HCl and ether for one hour. The ether layer was dried over sodium sulfate, filtered and evaporated to dryness *in vacuo*. A yellow viscous syrup was obtained which was dissolved in 25 ml of ethanol. Solids were formed, on refrigeration over-night, and were filtered off, washed with cold ethanol (4.2 g) and chromatographed on a column of 300 g of silica gel using a solvent mixture consisting of $CHCl_3$:$CH_3OH$:$CH_3CO_2H$ (200:10:2). The effluent was divided into fractions which were evaporated to dryness *in vacuo*. In this manner, 600 mg of a solid product consisting of the title compound, m.p. 192—194°C., were obtained.

Example 2

N-[N-[L-1-carboxy-2-(4-phenyl)phenylethyl]-L-phenylalanyl]-β-alanine

A suspension of 590 mg of the product from Example 1 in 100 ml of ethanol was shaken overnight with 200 mg of 10% Pd/C in a Parr apparatus. The resulting mixture was diluted with 40 ml of ethanol and 10 ml of water then heated on a steam bath briefly until all white precipitate dissolved. After cooling, the catalyst was filtered off. The filtrate was evaporated at 50°C under vacuum to 50 ml. The solid was filtered, washed with ethanol, and dried at room temperature under high vacuum for 6 hours to yield 155 mg of the title compound m.p. 226—228°C.

$[\alpha]_D 26 = 16.8$ (C=0.5, DMF).

Analysis Calculated: C, 70.40; H, 6.13; N, 6.08.

Found: C, 70.54; H, 6.26; N, 5.97.

Example 3

N-[N-[L-[1-[Pivaloyloxymethoxy[carbonyl[-2-phenylethyl)]-L-phenylalanyl]-β-alanine, pivaloyloxymethyl ester

A. N-(tert.-Butyloxycarbonyl)-β-alanine, pivaloyloxymethyl ester

Triethylamine (32.4 ml) is added to N-tert.-butyloxycarbonyl β-alanine (40 g) in N,N'-dimethylformamide at room temperature under a nitrogen atmosphere in a 1 l round bottom flask. The solution is stirred 15 minutes and chloromethyl pivalate (36.6 g) [M. Rasmussen & N.J. Leonard, J. Amer. Chem. Soc., *89*, 5439 (1967)] is added dropwise at 0°C. The solution is stirred at room temperature overnight. The mixture is then diluted with ethyl acetate, filtered, washed with water, then brine, and evaporated to give 70 g crude material which was chromatographed (silica gel) eluting with 15% ethyl acetate-hexane to give 62.5 g of product.

B. β-Alanine, pivaloyloxymethyl ester, trifluoroacetate

Trifluoroacetic acid (100 ml) is added to a solution of the product from example 3A (62 g) in methylene chloride (180 ml) at 0°C. The mixture is stirred at room temperature for two hours, and solvent removed in vacuum to give 100 g product as a pale yellow oil.

C. N-[L-1-(Phenylmethoxy))carbonyl-2-phenylethyl)]-L-phenylalanine

L-Phenylalanine benzyl ester hydrochloride, 190.4 g (0.652 mole) is suspended in 960 mls absolute methanol, 6.7 l dry 0.3 nm. (3Å sieves) tetrahydrofuran added and the slurry is stirred while adding about 50 ml triethylamine to bring the pH to 6.5—7.0. The pH is checked with EM Reagents ColorpHast indicator sticks, range pH 5—10, moistened with water before use. To the neutral slurry is added 200 g (0.98 mole) sodium phenyl pyruvate hydrate (Sigma), followed by 240 g crushed 0.3 nm (3Å) molecular sieves. (Sieves may be ground in a mortar and need not be finely powdered. If too fine, they are difficult to remove by filtration). The slurry is stirred at ambient temperature while adding a solution of 61.6 g (0.98 mole) sodium cyanoborohydride in 40 ml methanol plus 300 ml dry tetrahydrofuran dropwise over 5 hours. The reaction is stirred at ambient temperature for 48 to 72 hours while monitoring the disappearance of benzyl ester by thin layer chromatography. The reaction mixture is filtered to remove sieves, washing sieves well with hot methanol, as some product precipitates out on them.

The filtrate is concentrated on a rotary evaporator at 50°C to a syrup. This syrup is dissolved in 2.4 l ether in a 12 l round bottomed flask and stirred in an ice bath while adding 2.4 l 2.5% aqueous HCl. The large volume of HCN which is generated is passed into a sodium hydroxide trap. The mixture is allowed to stir for 2.5 to 3 hours while a white solid gradually forms at the interface and evolution of HCN stops. The 2-phase mixture is filtered (most of the aqueous phase may be drawn off in a separator before filtering as the product remains at the interface) and the solid is washed well with ether and dried in vacuo below 50°; yield 80—90 g., m.p. 175—180°C. This material is 90—95% pure L,L isomer as estimated by thin layer chromatography. The crude product is re-dissolved in about 10 l boiling, absolute methanol, some fine white inorganic insolubles filtered and the filtrate concentrated to 5 l at the boiling point, when flocculent white crystals appear. The product is allowed to cool slowly to room temperature and then to 0°C for 2—3 hours. The solid is collected and dried *in vacuo* at 50°C; yield wt 57—60 g, m.p. 185—186°C, $[\alpha]_D26$ 5.9 to 6.3°C* (DMSO, C=1). The product is greater than 98% L,L isomer as estimated by thin layer chromatography and high pressure liquid chromatography analysis. *(results of several runs).

D. N-(L-1-Benzyloxycarbonyl-2-phenylethyl)-L-phenylalanine pivaloyloxymethyl ester

A solution of 4.03 g (10 mmole) of the product from Example 3 C and 1.5 ml (11 mmole)triethylamine in 20 ml DMF is treated at ambient temperature with 1.6 ml (11 mmole) of chloromethyl-pivalate, stirred at 50—60°C for 24 hours, and the resulting slurry poured into water and extracted with 3 × 100 ml ether. Some insolubles are filtered off, and the ether phase washed with water, dried and concentrated to an oil; yield 4.8 g; NMR consistent with structure of title compound.

E. N-(L-Carboxy-2-phenylethyl)-L-phenylalanine, pivaloyloxymethyl ester

The crude diester from Example 3D (4.8 g) is hydrogenated in a Parr apparatus at 3.45 bar (60 psig) in 50 ml methanol + 5 ml $H_2O$ in the presence of 0.4 g 10% Pd/C for 3 hours. The resulting reaction mixture is filtered and concentrated to a damp solid which was re-crystallized from methanol/$H_2O$. White fluffy needles are filtered off and dried *in vacuo*; yield 3.0 g, m.p. 122—124°C. TLC showed essentially one spot, $R_f$ 0.2, in $CHCl_3$/MeOH/HOAc 100: 1:0.0.5.

F. N-(L-1-Pivaloyloxymethylcarbonyl-2-phenylethyl)-L-phenyl-alanine-β-alanine, pivaloyloxymethyl ester

A mixture of 1.0 g (2.3 mmole) of the product from Example 3E and 0.89 g (2.3 mmole) of the product from Example 3B in 25 ml DMF is treated with 1.01 ml (8 mmole) N-ethyl-morpholine followed by 352 mg (2.3 mmole) 1-hydroxybenzotriazole hydrate and 439 mg (2.3 mmole) 1-(3-dimethylaminopropyl)-3-ethyl carbodiimide hydrochloride, and allowed to stir at ambient temperature overnight. Thin layer chromatography still shows starting material present. The reaction mixture is heated to 40—50°C., for 6 hours and allowed to stand at room temperature overnight. The mixture is poured into water and extracted with several portions of ether, then the ether phase is washed well with water, dried and concentrated to an

oil (1.2 g). The oil is chromatographed on Merck thin layer chromatography grade silica gel 60—G to yield 0.9 g of oil showing a single spot, $R_f$ 0.4 in EtOAc/Hexane 1:2 (same system as used for column chromatography.)

Analysis: Calculated for $C_{33}H_{44}N_2O_9$: C, 64.69; H, 7.24; N, 4.57.

Found: C, 64.47; H, 7.20; N, 4.29.

$[\alpha]_D26$ −21.7° (DMF, C=1.0).

Example 4

N-[N-[L-[1-[(2,2-dimethyl-1-oxopropoxy)methoxy]carbonyl]-2-(4-phenyl)-phenylethyl]-L-phenylalanyl]-β-alanine

A. N-[D,L-1-Carboxy-2-(4-phenyl)phenylethyl]-L-phenylalanine, benzyl ester.

A stirred suspension of 4-phenylphenylpyruvic acid (24.0 g) and L-phenylalanine benzyl ester hydrochloride (23.2 g) in 1 l of THF/ethanol (9:1) was brought to pH 6.6 by gradual addition of triethylamine. In the course of this process, all solids dissolved. After stirring the resulting solution for 2 hours at room temperature, a solution of sodium cyanoborohydride (3.5 g) in the same solvent was added dropwise with stirring. The reaction mixture was allowed to stir overnight at room temperature.

The reaction mixture was then concentrated to 200 ml under reduced pressure. The residue was poured into 600 ml of 0.3N HCl with cooling and stirring. A gummy solid separated. The aqueous material was decanted off, and the remaining solid stirred with 120 ml of ethanol. The resulting solids were filtered, and the wet solids stirred with 100 ml of fresh ethanol. After standing overnight, the solids were filtered and dried to give 22.3 g of solid product.

B. N-[D,L-[1.[(2,2-Dimethyl-1-oxopropoxy)methoxy]carbonyl]-2-(4-phenyl)phenylethyl]-L-phenylalanine, benzyl ester.

Triethylamine (3.05 ml) was added to a solution of N-[D,L-1-carboxy-2-(4-phenyl)phenylethyl]-L-phenylalanine, benzyl ester (9.6 g) in 30 ml of dimethylformamide. The mixture was stirred at room temperature for 20 min. and chloromethyl pivalate (3.15 ml) was added. The resulting mixture was heated in a bath at 45—55°C for 4 hours with stirring, then allowed to stir at room temperature overnight. The resulting mixture was diluted with 300 ml of water and extracted with three 150 ml portions of ether. The combined ether layers were extracted twice with 100 ml portions of water, and the ether solution dried over anhydrous $Na_2SO_4$. Filtration and evaporation in vacuo gave 7.5 g of syrupy product.

This material was chromatographed on 135 g of silica gel eluting with a mixture of ethylacetate-cyclohexane (85:15). Fractions containing the desired diastereomeric mixture of products were identified by thin-layer chromatography, combined, and evaporated to dryness in vacuo to give 5.9 g of product as a syrup.

C. N-[D,L-[1-[(2,2-Dimethyl-1-oxopropoxy)methoxy]carbonyl]-2-(4-phenyl)phenylethyl]-1-phenylalanine

A solution of the above product (5.9 g) in 175 ml of ethanol was hydrogenated at 1.03—2.07 bar 15—30 psig over 750 mg of 10% Pd/C for 2 hours. The reaction mixture was diluted with an additional 250 ml of ethanol and warmed to 45°C to dissolve the precipitated product. Catalyst was filtered from the warm solution, and the filtrate evaporated to give a total of 4.4 g product.

D. N-[N-[L-[1-[(2,2-Dimethyl-1-oxopropoxy)methoxy]carbonyl]-2-(4-phenyl)phenylethyl]-L-phenylalanyl]-β-alanine, benzyl ester.

N-ethylmorpholine (1.2 ml) was added to a stirred mixture of the above product (3.25 g), N-(N,N-dimethylaminopropyl)-N'-ethylcarbodiimide hydrochloride (1.7 g), of hydroxybenzotriazole (1.3 g) and β-alanine benzyl ester p-toluenesulfonate (3.0 g) in 25 ml of dimethylformamide. The mixture was stirred at room temperature for 3 hours, diluted with 200 ml of ice-water and extracted with two 125 ml portions of ether. The combined extracts were washed with 250 ml of water and dried over anhydrous $MgSO_4$. Filtration and evaporation gave 5.25 g of residue. Thin-layer chromatography (silica gel, $CHCl_3$/EtOAc — 10:1) showed two major products, $R_f$=0.36 and $R_f$=0.32 (partially overlapping). This material was chromatographed on 350g of silica gel (thin-layer chromatography grade), eluting with $CHCl_3$-EtOAc (100:5). Fractions containing the pure individual components were identified by thin layer chromatography, combined and evaporated. In this manner 650 mg of faster moving component (L,L-diastereoisomer) was obtained along with 590 mg of L,D-diastereoisomer.

The final product was obtained by hydrogenating a solution of 650 mg of L, L-diastereoisomer above in 50 ml ethanol over 50 mg of 10% Pd/C at 1.03—3.10 bar (15—45 psig) for 3 hours. Catalyst was filtered off, and the filtrate evaporated to dryness in vacuo at 30°C. The residue was chromatographed on 50 g of t.l.c. grade silica gel, eluting initially with 300ml of $CHCl_3$/EtOAc (10:1), then with $CHCl_3$/$CH_3OH$/AcOH (600:10:2). Fractions containing pure product were identified by thin layer chromatography (silica gel) $CHCl_3$/$CH_3OH$/AcOH (600:10:2), $R_f$ = 0.31. These fractions were combined, evaporated, and the residue dried at room temperature in high vacuum overnight. The residue was recrystallized from ether and the product dried at 45°C for 3 1/2 hours in high vacuum. Obtain 250 mg solid, m.p. 101—103°C, $[\alpha]_D26$ −22° (c = 0.5, DMF).

Example 5

N-[N-[L-[1-(2,2-dimethyl-1-oxopropoxy)methoxy]carbonyl]-2-phenylethyl]-L-phenylalanyl]-β-alanine

A mixture of 0.9 g (2.1 mmole) of N-(L-1-carboxy-2-phenylethyl)-L-phenylalanine, pivaloyloxymethyl ester (Example 3E) and equimolar amounts of β-alanine benzyl ester p-toluenesulfonate (73) mg), 1-hydroxybenzotriazole hydrate (321 mg) and 1-(3-dimethylaminopropyl)-3-ethyl-carbodiimide HCl (401 mg) were dissolved in 20 ml DMF containing 0.8 ml (6.3 mmole) N-ethylmorpholine and the mixture stirred at ambient temperature overnight.

The yellow solution was poured into water (150 ml) and extracted with 3 × 100ml ether. The ether phase was washed with water, dried and concentrated to an oil, 1.1 g showing one major spot with minor impurity on thin-layer chromatography.

The total product was chromatographed on 150 g silica gel eluting with ethyl acetate/hexane (30:70). The fractions were combined and concentrated to an oil, $[\alpha]_D 26$ — 20.0° (DMF, c = 1).

The above-described product (1.5 g) was hydrogenated at 40 psig in 100 ml absolute EtOH over 0.2 g 10% Pd/C for 3 hours.

The catalyst was filtered and the filtrate concentrated to an oil which crystallized, upon drying in high vacuum, overnight. On standing under hexane fine colourless crystals were obtained, which on filtration and drying gave 1.2 g of product, m.p. 93—95°C, $[\alpha]_D 26$ — 27.3° (DMF, c = 1).

By following the procedures described in the preceding description and examples, using appropriate intermediates, each of the specific compounds 1—63 listed above can be prepared.

In the following table, the shorthand notations $C_6H_5CH_2$ and $C_7H_7$ indicate the benzyl groups; and $C_6H_5$ indicates the phenyl group. By way of example utilizing the above described methods, the following compounds of the invention were prepared. Except where indicated all compounds have the L absolute stereochemistry at any chiral centre, and $R_5$ equal to hydrogen.

$R_1\ C^*HCOR_2\ NH\ C^*HR_3\ CONH(CH_2)_p\ C^*R_4R_5\ COR_6$

| Compound | R 1 | R 2 | R 3 | R 4 | R 6 | p | m.p. °C | $[\alpha]_D^0$ (C, Solv.) |
|---|---|---|---|---|---|---|---|---|
| A | $C_6H_5CH_2$— | —$OCH_2C_6H_5$ | $C_7H_7$— | H | —$OCH_2OCOC(CH_3)_3$ | 1 | oil | −25.0 (0.5, MeOH) |
| B | $C_6H_5CH_2$— | —$OCH_2OCOC(CH_3)_3$ | $C_7H_7$— | H | —$OCH_2OCOC(CH_3)_3$ | 1 | oil | −21.7 (1.0, DMF) |
| C | $C_6H_5CH_2$— | —$OCH_2OCOC(CH_3)_2$ | $C_7H_7$— | H | —$OCH_2CH_3$ | 1 | oil | −3.8 (1.0, DMF) |
| D | $C_6H_5CH_2$— | —$OCH_2OCOC(CH_3)_3$ | $C_7H_7$— | H | —$OCH_2C_6H_5$ | 1 | oil | −20.0 (1.0, DMF) |
| E | $C_6H_5CH_2$— | OH | $C_7H_7$— | H | —$O(CH_2)_2O$—$C_6H_5$ | 1 | 141—4 | −5.7 (0.4, MeOH) |
| F | $C_6H_5CH_2$— | OH | $C_7H_7$— | H | —$OCH_2OCOC(CH_3)_3$ | 1 | 180—1 | — |
| G[a] | $C_6H_5CH_2$— | OH | $C_7H_7$— | $CH_3$ | OH | 1 | 224—6 | — |
| H[a] | $C_6H_5CH_2$— | —$OCH_2C_6H_5$ | $C_7H_7$— | $CH_3$ | —$OCH_2OCOC(CH_3)_3$ | 1 | oil | — |
| I[a] | $C_6H_5CH_2$— | OH | $C_7H_7$— | —$CH_2C_6H_5$ | OH | 1 | 235—7 | −23.7 (1.0, DMF) |
| J | p—$C_6H_5$—$C_6H_4CH_2$— | OH | $C_7H_7$— | H | OH | 1 | 226—8 | −16.8 (0.5 DMF) |
| K | $C_6H_5CH_2$— | OH | p—$C_6H_5$—$C_6H_4CH_2$ | H | OH | 1 | 229—30 | −7.5 (0.5, DMF) |
| L | $C_6H_5CH_2$— | —$NHC_6H_4$—o—$CH_3$ | $C_7H_7$ | H | —$OCH_2OCOC(CH_3)_3$ | 1 | amorphous | — |
| M | $C_6H_5CH_2$— | OH | $(CH_3)_2CHCH_2$ | $C_7H_7$ | OH | 0 | 178—80 | — |
| AM[b] | $C_6H_5CH_2$— | OH | $(CH_3)_2CHCH_2$ | $C_7H_7$ | OH | 0 | 191—92 | — |
| N | $C_6H_5CH_2$— | OH | $C_7H_7$ | $C_7H_7$ | OH | 0 | | |
| O | $C_6H_5CH_2SCH_2$ | OH | $C_7H_7$ | $CH_3$ | OH | 0 | 152—4 | |

[b] D stereochemistry at chiral centre bonded to $R_1$
[a] 1:1 mixture of DαL stereoisomers

The compounds having structural formula I inhibit the activity of enzymes designated enkephalinases. The compounds are particularly useful for the inhibition of enkephalinase A, which is derived from the striata of both rats and humans. In *in vitro* tests, selected compounds having structural formula I in a concentration range from $10^{-9}$ to $10^{-6}$M have been found to inhibit the activity of the aforementioned enzyme by 50% or more.

The following test procedure was utilized to assay the enkephalinase A inhibition of the compounds having structural formula I.

Enkephalin (ENK) degrading activity was separated into the following three fractions according to the method of Gorenstein and Snyder, [Life Sci., *25,* 2065 (1979): Enk'ase A ($Gly^{3}$-$Phe^{4}$), Aminopeptidase, (AP) ($Tyr^{1}$-$Gly^{2}$), and Enk'ase B ($Gly^{2}$-$Gly^{3}$).

Enzyme activity was separated by taking the brain tissue (minus cerebellum) from Sprague-Dawley rats and homogenizing it in 30 volumes of 50 mM Tris buffer, pH 7.4, using a Brinkmann Polytron. The resulting homogenate is centrifuged at 50,000xg for 15 min. The pellet, constituting the membrane bound enzyme material, is washed by re-suspending it in Tris and re-centrifuging it 4 times.

Following washing, solubilization of the membrane pellet is achieved by incubating it for 45 min. at 37°C in the presence of 15 volumes (based on initial brain weight) of 50 mM Tris-1% Triton® X—100 buffer, pH 7.4. After centrifugation at 100,000xg for 60 minutes to remove non-solubilized material, the Triton® soluble supernatant is layered on a 1.5 × 30 cm DEAE Sephacel column previously equilibrated with 50 mM Tris-0.1% Triton®, pH 7.4. Material is eluted from the column using a 1 liter linear NaCl gradient running from 0.0 to 0.4 M. Eluant is collected in 7 ml fractions, each of which is assayed for enkephalin degrading activity. Under these conditions Enk'ase A activity is found to elute between 120 and 200 ml followed by AP activity (260 to 400 ml) and finally by Enk'ase B activity between 420 and 450 ml.

Enkephalin degrading activity is monitored using a radiometric assay. The substrate is $^{3}H$-$Met_{a}{}^{5}$ENK (50.1 Ci/mmol, New England Nuclear) diluted to 0.05 M Tris buffer, pH 7.4, such that the final reaction mixture concentration is 40 nM. Total reaction mixture volume including enzyme and substrate is 250 yl. Incubation is carried out for 90 min at 37°C. To stop the reaction, tubes are transferred to a boiling water bath for 15 min.

Assay products are separated from one another using thin layer chromatography. A 4 µl aliquot of the reaction mixture is spotted on a Baker-flex Silica Gel 1B plate (20 × 20 cm) along with unlabeled standards ($Met^{5}$-ENK, tyrosin, tyrosylglycine, tyrosyl-glycyl-glycine) and the components co-chromatographed in an isopropanyl: ethyl acetate: 5% acetic acid solvent system (2:2:1) which is capable of resolving $Met^{5}$-ENK from its breakdown products. Total running time is approximately 17 hours. TLC tanks are gassed with nitrogen prior to starting the run. Following the run, markers are visualized with ninhydrin spray. These spots, along with remaining plate regions, are cut from the plate and the radioactivity corresponding to each monitored using liquid scintillation counting. $IC_{50's}$ are determined using liner regression techniques.

Utilizing this procedure, the following nanomolar (nM) concentrations for the specified compounds were found to inhibit the action of enkephalinase A by 50% ($IC_{50}$).

TABLE A

| Compounds | Enkephalinase A Inhibition $IC_{50}$ nM |
|---|---|
| A | N.A.[1] |
| B | N.A.[1] |
| C | N.A.[1] |
| D | N.A.[1] |
| E | 230 |
| F | 580 |
| G | 6.4 |
| H | N.jA.[1] |
| I | 6.3 |
| J | 2.5 |
| K | 0.9 |
| L | N.A.[1] |
| M | 140 |
| AM | 12 |
| N | 60 |
| O | 170 |

[1]No activity at $<10_5$nM

The following test procedure was utilized to assess the noted compounds' potentiation of the analgesic effects of (DAla$^2$-Met$^5$)-enkephalinamide (DAEAM). Background for the use of this procedure is given in Chipkin, R.E., Iorio, L.C., Barnett, A., Berger, J., and Billard, W., *Regulatory Peptides: From Molecular Biology to Function* edited by E. Costa and M. Trabucchi, Raven Press, New York, 1982, pp. 235—242.

Male CFI mice (19—23 g) from Charles River Breeding Labs, Mass., are used (N=10/dose or dose combination). Tailflick testing is done similar to that of Dewey and Harris, *Method in Narcotic Research*, Eds., S. Ehrenpreis and A. Neidle, pp. 101—109, Marcel Dekker, Inc., New York, 1975 using a radiant heat noxious stimulus. Following determination of control latencies (typically 2—3 sec), the mice are first injected (sc or po) with either vehicle or drug and after an appropriate interval injected intracerebroventricularly (icv) with either vehicle (10 µl of saline) or DAEAM according to Haley and McCormic, *Br.,* J. *Pharmacol, 12,* 12 (1957). Tail flick latencies are re-determined 30 min. later, as this has previously been determined to be the time of peak analgesia for DAEAM, a cut-off of 10 seconds is employed.

Utilizing this procedure, the following $ED_{50}$ values (the dose at which half the test animals displayed analgesia) were obtained for selected compounds.

TABLE B

| Compound | DAEAM Potentatiation $ED_{50}$ (route) |
|---|---|
| A | 155 mg/k (po) |
| B | 37 mg/k (po) |
| C | 100 mg/k (po) |
| D | 100 mg/k (po) |
| E | 30—60 mg/k (po) |
| F | 100 mg/k (sc)[2] |
| G | 50 µg (icv) |
| H | 100 mg/k (po) |
| I | 50 µg (icv)[2] |
| K | 50 µg (icv)[2] |
| L | 100 mg/k (po) |

[2] ED100

It should be noted that compounds A, B, C, F and H are ester derivatives; compound H being an ester derivative of compound G, and compound L is an amide derivative.

Such derivatization is found to confer oral activity to the parent entity which show poor absorption from the gastrointestinal tract. These derivatives, which show no activity *in vitro* at $10^5$nM (see Table A), are bioactivated *in vivo* to deliver the parent (*in vivo* enkephalinase A inhibitors) to a site of action within the central nervous system (see Table B).

**Claims for the Contracting States: BE CH DE FR GB IT LI LU NL SE**

1. A compound having the formula I

$$R_1C^*(HCOR_2)—NH—C^*(HR_3)—CONH(CH_2)_p—C^*(R_4R_5)—COR_6$$

or a mixture of enantiomers or diasterioisomers containing the same, and the pharmaceutically acceptable salts thereof wherein:

$R_1$ is alkyl having from 1 to 6 carbon atoms, adamantylmethyl, cycloalkylmethyl having from 4 to 8 carbon atoms or $A—X_m—C_nH_{2n}—$ wherein X is oxygen or sulfur, A is phenyl which may be substituted with the group Y, wherein Y is halogen, hydroxy, trifluoromethyl; alkoxy having from 1 to 6 carbon atoms, alkyl having from 1 to 6 carbon atoms, 2- or 3-furanyl, 2- or 3-thienyl, or phenyl (which may be substituted with halogen, hydroxy, trifluoromethyl, alkoxy having from 1 to 6 carbon atoms or alkyl having from 1 to 6 carbon atoms, or may also be benzyl (the phenyl ring of which may be substituted with the group Y, as defined above), 1- or 2-naphthyl, 2- or 3-furanyl or 2- or 3-thienyl; m is 0 or 1 and n is 0, 1, 2, 3, or 4;

$R_2$ and $R_6$ may be the same or different and are hydroxy, alkoxy having from 1 to 8 carbon atoms, $B—X_m—C_nH_{2n}—O—$ wherein B is phenyl (which may be substituted with the group Y, as defined herein) or 1- or 2-naphthyl, X, m, and n are as defined herein provided that when n = 0, m = 0, $—OCH_2OCO$-alkyl in which the alkyl group has 1 to 6 carbon atoms, $—OCH_2CO$-phenyl (the phenyl ring of which may be substituted with the group Y, as defined above), 1-glyceryl,

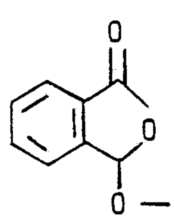

or

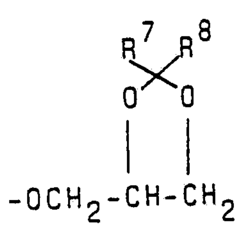

wherein $R_7$ is hydrogen, alkyl having from 1 to 6 carbon atoms, or phenyl which may be substituted with the group Y, as defined above, and $R_8$ is hydrogen or alkyl having from 1 to 6 carbon atoms;

$R_2$ may also be $—NR_7R_8$ wherein $R_7$ and $R_8$ are as defined above;

$R_3$ is alkyl having from 1 to 6 carbon atoms, cycloalkylmethyl having from 4 to 8 carbon atoms, 2- or 3-thienylmethyl, 2- or 3-furanylmethyl, 1- or 2-naphthylmethyl, or benzyl the phenyl ring of which may be substituted with the group Y, as defined above;

$R_4$ is $D—C_nH_{2n}—O_m—$ wherein D is hydrogen, alkyl having from 1 to 4 carbon atoms or phenyl which may be substituted with the group Z, wherein Z is halogen, hydroxy, trifluoromethyl, alkoxy having from 1 to 6 carbon atoms, or alkyl having from 1 to 6 carbon atoms;

m and n are as defined above;

$R_5$ is hydrogen or alkyl having from 1 to 6 carbon atoms; and p is 0, 1 or 2;
subject to the following provisos

(i) that when $R_4$ and $R_5$ are both hydrogen, and p is 1 or 2 then;

a) $R_1$ is chosen from adamantyl, 4-phenylphenylethyl, $A—X_m—C_nH_{2n}—$ wherein m is 1, X is as defined above and *n* is 1, 2, 3, or 4, 1- or 2-naphthyl, 2- or 3-furanyl and 2- or 3-thienyl; and/or

b) $R_2$ and/or $R_6$ are chosen from $B—Xm—CNH_{2n}—O—$ wherein B is as defined above, m is one, X is as defined above and n is 1, 2, 3 or 4, $—OCH_2OCO$-alkyl in which the alkyl group has 1 to 6 carbon atoms, $—OCH_2CO$-phenyl (in which the phenyl group may be substituted by the group Y, as defined herein), 1-glyceryl,

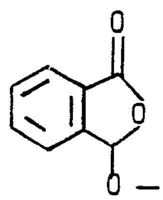 or 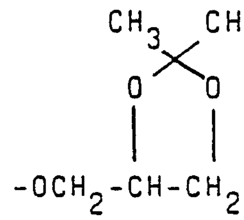 .

and $R_2$ may also be $—NR_7R_8$ wherein $R_7$ and $R_8$ are as defined herein; and/or

c) $R_3$ is 2- or 3-furanylmethyl or 4-phenylphenylethyl;

(ii) when P is zero then

$R_1$ is benzyl or benzylthiomethyl;

$R_3$ is benzyl or maybe leucyl when $R_1$ is benzyl;

$R_4$ is benzyl when $R_1$ is benzyl, and is methyl when $R_1$ is benzylthiomethyl;

$R_5$ is hydrogen.

2. A compound of formula I as defined in claim 1, characterized in that $R_4$ represents a group $D—C_nH_{2n}—O_m—$ as defined in claim 1 in which the sum of n and m is at least one.

3. A compound of formula I as defined in claim 1 or 2, characterized in that one of $R_2$ and $R_6$ is 2-phenoxyethoxy, 1-glyceryl,

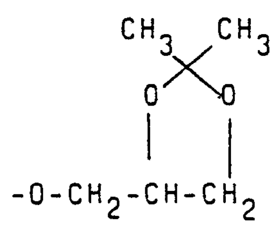 , 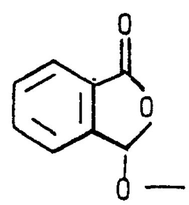 ,

or (2,2-dimethyl-1-oxopropoxy)methoxy, and the other is chosen from the groups defined above or is hydroxy, methoxy, ethoxy or benzyloxy.

4. A compound as defined in claim 2, characterized in that

$R_1$ is benzyl optionally *para* substituted by chlorine, methoxy, methyl, or phenyl, 2-phenylethyl or 1- or 2-naphthylmethyl,

$R_2$ and $R_6$ are the same or different and are hydroxy, methoxy, ethoxy, benzyloxy, 2-phenoxyethoxy, 1-glyceryl,

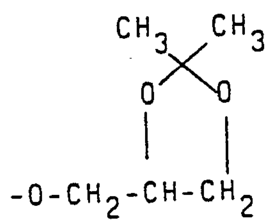 , 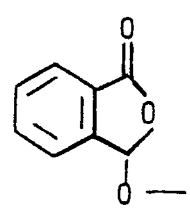 ,

or (2,2-dimethyl-1-oxopropoxy)methoxy, and

$R_3$ is benzyl, p-methylbenzyl, p-phenylbenzyl, 1-naphthylmethyl or 3-thienylmethyl.

5. A compound as defined in claim 4, characterized in that $R_4$ is methyl or benzyl and $R_5$ is hydrogen.

6. A compound as defined in claim 3, characterized in that $R_1$ and $R_3$ are as defined in claim 4, $R_4$ is hydrogen, methyl or benzyl, and $R_5$ is hydrogen.

7. A compound as defined in claim 6, characterized in that $R_4$ is hydrogen.

8. A compound as defined in any one of the preceding claims in which p is one.

9. N-[N-[L-1-(2,2-dimethyl-1-oxopropoxy)methoxy]carbonyl]-2-phenylethyl]-L-phenylalanyl]-ß-alanine, (2,2-dimethyl-1-oxopropoxy)methyl ester;
N-[N-[L-1-[phenylmethoxy]carbonyl]-2-phenylethyl]-L-phenylalanyl]-β-alanine, (2,2-dimethyl-1-oxopropoxy)methyl ester;
N-[N-[L-1-carboxy-2-(4-phenyl)phenylethyl]-L-phenylalanyl]-β-alanine;
N-[N-[L-[1-[(2,2-dimethyl-1-oxopropoxy)methoxy]carbonyl]-2-(4-phenyl)phenylethyl]-L-phenylalanyl]-β-alanine, (2,2-dimethyl-1-oxopropoxy)methyl ester;
N-[N-[L-1-carboxy-2-phenylethyl]-L-(4-phenyl)phenylalanyl]-β-alanine;
N-[N-[L-[1-[(2,2-dimethyl-1-oxopropoxy)methoxy]carbonyl]-2-(4-phenyl)phenylethyl]-L-(4-phenyl)phenylalanyl-β-alanine, (2,2-dimethyl-1-oxopropoxy)methyl ester;
N-[N-[L-[1-[(2,2-dimethyl-1-oxopropoxy)methoxy]carbonyl]-2-phenylethyl]-L-(4-phenyl)phenylalanyl]-β-alanine, (2,2-dimethyl-1-oxopropoxy)methyl ester;
N-[N-[L-1-carboxy-2-phenylethyl]-L-phenylalanyl]-L-[α-methyl)-β-alanine;
N-[N-[L-[1-[(2,2-dimethyl-1-oxopropoxy)methoxy]carbonyl]-2-phenylethyl]-L-phenylalanyl]-L-(α-methyl)-β-alanine, (2,2-dimethyl-1-oxopropoxy)methyl ester;
N-[N-[(L-1-carboxy-2-phenylethyl)]-L-phenylalanyl]-β-alanine, 2-phenoxyethyl ester;
N-[N-[L-[1-[(2,2-dimethyl-1-oxopropoxy)methoxy]carbonyl]-2-phenylethyl]-L-phenylalanyl]-β-alanine;
N-[N-[L-[1-[(2-phenoxy)ethoxy]carbonyl]-2-phenylethyl]-L-phenylalanyl]-β-alanine;
N-[N-[L-[1-[(1-oxo-3-isobenzofuranyloxy)]carbonyl]-2-phenylethyl]-L-phenylalanyl-β-alanine;
N-[N-[L-[1-[(2,3-dihyroxy-1-propoxy]carbonyl]-2-phenylethyl]-L-phenylalanyl]-β-alanine;
N-[N-[L-[1-[(2,2-dimethyl-1,3-dioxolan-4-yl)methoxy]carbonyl]-2-phenylethyl]-L-phenylananyl)-β-alanine;
N-[N-[L-[1-[(2,2-dimethyl-1-oxopropoxy)methoxy]carbonyl]-2-(4-phenyl)phenylethyl]-L-phenylalanyl]-β-alanine;
N-[N-[L-[1-[(2-phenoxy)ethoxy]carbonyl]-2-(4-phenyl)phenylethyl]-L-phenylalanyl]-β-alanine;
N-[N-[L-[1-[(1-oxo-3-isobenzofuranyloxy)]carbonyl]-2-(4-phenyl)phenylethyl]-L-phenylalanyl]-β-alanine;
N-[N-[L-[1-[(2,3-dihydroxy)-1-propoxy]carbonyl]-2-(4-phenyl)phenylethyl]-L-phenylalanyl]-β-alanine;
N-[N-[L-[1-[(2,2-dimethyl-1,3-dioxolan-4-yl)methoxy]carbonyl]-2-(4-phenyl)phenylethyl]-L-phenylalanyl]-β-alanine;
N-[N-[L-[1-[(2,2-dimethyl-1-oxopropoxy)methoxy]carbonyl]-2-phenylethyl]-L-(4-phenyl)phenylalanyl]-β-alanine;
N-[N-[L-[1-[(2-phenoxy)ethoxy]carbonyl]-2-phenylethyl]-L-(4-phenyl)phenylalanyl]-β-alanine;
N-[N-[L-[1-[(1-oxo-3-isobenzofuranyloxy)]carbonyl]-2-phenylethyl]-L-(4-phenyl)phenylalanyl]-β-alanine;
N-[N-[L-[1-[(2,3-dihydroxy)-1-propoxy]carbonyl]-2-phenylethyl]-L-(4-phenyl)phenylalanyl]-β-alanine;
N-[N-[L-[1-[(2,2-dimethyl-1,3-dioxolan-4-yl)methoxy]carbonyl]-2-phenylethyl]-L-(4-phenyl)phenylalanyl]-β-alanine;
N-[N-[L-[1-[(2,2-dimethyl-1-oxopropoxy)methoxy]carbonyl]-2-(4-phenyl)phenylethyl]-L-(4-phenyl)phenylalanyl]-β-alanine;
N-[N-[L-[1-[(2-phenoxy)ethoxy]carbonyl]-2-(4-phenyl)phenylethyl]-L-(4-phenyl)phenylalanyl]-β-alanine;
N-[N-[L-[1-[(1-oxo-3-isobenzofuranyloxy)]carbonyl]-2-(4-phenyl)phenylethyl]-L-(4-phenyl)phenylalanyl]-β-alanine;
N-[N-[L-[1-[(2,3-dihydroxy)-1-propoxy)carbonyl]-2-(4-phenyl)phenylethyl]-L-(4-phenyl)phenylalanyl]-β-alanine;
N-[N-[L-[1-[(2,2-dimethyl-1,3-dioxolan-4-yl)methoxy]carbonyl]-2-(4-phenyl)phenylethyl]-L-(4-phenyl)phenylalanyl]-β-alanine;
N-[N-[L-[1-[(2,2-dimethyl-1-oxopropoxy)methoxy]carbonyl]-2-phenylethyl]-L-phenylalanyl]-D,L-α-methyl-β-alanine;
N-[N-[L-[1-[(2-phenoxy)ethoxy]carbonyl]-2-phenylethyl]-L-phenylalanyl]-D,L-α-methyl-β-alanine;
N-[N-[L-[1-[(1-oxo-3-isobenzofuranyloxy)]carbonyl]-2-phenylethyl]-L-phenylalanyl]-D,L-α-methyl-β-alanine;
N-[N-[L-[1-[(2,3-dihydroxy)-1-propoxy]carbonyl]-2-phenylethyl]-L-phenylalanyl]-D,L-α-methyl-β-alanine;
N-[N-[L-[1-[(2,2-dimethyl-1,3-dioxolan-4-yl)methoxy]carbonyl]-2-phenylethyl]-L-phenylalanyl]-D,L-α-methyl-β-alanine;
N-[N-[L-[1-[(2,2-dimethyl-1-oxopropoxy)methoxy]carbonyl]-2-(4-phenyl)phenylethyl]-L-phenylalanyl]-D,L-α-methyl-β-alanine;
N-[N-[L-[1-[(2-phenoxy)ethoxy]carbonyl]-2-(4-phenyl)phenylethyl]-L-phenylalanyl]-D,L-α-methyl-β-alanine;
N-[N-[L-[1-[(1-oxo-3-isobenzofuranyloxy)]carbonyl]-2-(4-phenyl)phenylethyl]-L-phenylalanyl]-D,L-α-methyl-β-alanine;
N-[N-[L-[1-[(2,3-dihydroxy)-1-propoxy]carbonyl]-2-(4-phenyl)phenylethyl]-L-phenylalanyl]-D,L-α-methyl-β-alanine;

N-[N-[L-[1-[(2,2-dimethyl-1,3-dioxolan-4-yl)methoxy]carbonyl]-2-(4-phenyl)phenylethyl]-L-phenylalanyl]-D,L-α-methyl-β-alanine;

N-[N-[L-[1-[(2,2-dimethyl-1-oxopropoxy)methoxy]carbonyl]-2-phenylethyl]-L-(4-phenyl)phenylalanyl]-D,L-α-methyl-β-alanine;

N-[N-[L-[1-[(2-phenoxy)ethoxy]carbonyl]-2-phenylethyl]-L-(4-phenyl)phenylalanyl]-D,L-α-methyl-β-alanine;

N-[N-[L-[1-[(1-oxo-3-isobenzofuranyloxy)]carbonyl]-2-phenylethyl]-L-(4-phenyl)phenylalanyl]-D,L-α-methyl-β-alanine;

N-[N-[L-[1-[(2,3-dihydroxy)-1-propoxy]carbonyl]-2-phenylethyl]-L-(4-phenyl)phenylalanyl]-D,L-α-methyl-β-alanine;

N-[N-[L-[1-[(2,2-dimethyl-1,3-dioxolan-4-yl)methoxy]carbonyl]-2-phenylethyl]-L-(4-phenyl)phenylalanyl]-D,L-α-methyl-β-alanine;

N-[N-[L-[1-[(2,2-dimethyl-1-oxopropoxy)methoxy]carbonyl]-2-(4-phenyl)phenylethyl]-L-(4-phenyl)phenylalanyl]-D,L-α-methyl-β-alanine;

N-[N-[L-[1-[(2-phenoxy)ethoxy]carbonyl]-2-(4-phenyl)phenylethyl]-L-(4-phenyl)phenylalanyl]-D,L-α-methyl-β-alanine;

N-[N-[L-[1-[(1-oxo-3-isobenzofuranyloxy)]carbonyl]-2-(4-phenyl)phenylethyl]-L-(4-phenyl)phenylalanyl]-D,L-α-methyl-β-alanine;

N-[N-[L-[1-[(2,3-dihydroxy)-1-propoxy]carbonyl]-2-(4-phenyl)phenylethyl]-L-(4-phenyl)phenylalanyl]-D,L-α-methyl-β-alanine;

N-[N-[L-[1-[(2,2-dimethyl-1,3-dioxolan-4-yl]methoxy]carbonyl]-2-(4-phenyl)phenylethyl]-L-(4-phenyl)phenylalanyl]-D,L-α-methyl-β-alanine;

N-[N-[L-[1-[(2,2-dimethyl-1-oxopropoxy)methoxy]carbonyl]-2-phenylethyl]-L-2-thienylalanyl]-β-alanine;

N-[N-[L-[1-[(2,2-dimethyl-1-oxopropoxy)methoxy]carbonyl]-2-phenylethyl]-L-3-thienylalanyl]-β-alanine;

N-[N-[L-[1-[(2,2-dimethyl-1-oxopropoxy)methoxy]carbonyl]-2-phenylethyl]-L-2-furoalanyl]-β-alanine;

N-[N-[L-[1-[(2,2-dimethyl-1-oxopropoxy)methoxy]carbonyl]-2-phenylethyl]-L-phenylalanyl]-α-methylalanine;

N-[N-[L-[1-[(2,2-dimethyl-1-oxopropoxy)methoxy]carbonyl]-2-phenylethyl]-L-phenylalanyl]-L-α-hydroxy-β-alanine;

N-[N-[L-[1-[(2,2-dimethyl-1-oxopropoxy)methoxy]carbonyl]-2-phenylethyl]-L-phenylalanyl]-D-α-hydroxy-β-alanine;

N-[N-[L-[1-[(2,2-dimethyl-1-oxopropoxy)methoxy]carbonyl]-2-phenylethyl]-L-phenylalanyl]-L-α-methoxy-β-alanine;

N-[N-[L-[1-[(2,2-dimethyl-1-oxopropoxy)methoxy]carbonyl]-2-phenylethyl]-L-phenylalanyl]-D-α-methoxy-β-alanine;

N-[N-[L-[1-[(2,2-dimethyl-1-oxopropoxy)methoxy]carbonyl]-2-phenylethyl]-L-phenylalanyl]-D,L-α-methyl-β-alanine benzyl ester;

N-[N-[L-[1-[(2,2-dimethyl-1-oxopropoxy)methoxy]carbonyl]-2-(4-methoxy)phenylethyl]-L-phenylalanyl-β-alanine;

N-[(L)-1-carboxy-2-phenylethyl]-L-phenyl alanyl-L-phenylalanine;

N-[(L)-1-carboxy-2-phenylethyl]-L-leucyl-L-phenylalanine; and/or

a salt of one of the foregoing, which contains a free acid function, with a pharmaceutically acceptable base, or a salt of one of the foregoing with a pharmaceutically acceptable acid.

10. A composition which comprises a compound as defined in any one of the preceding claims in combination with a pharmaceutically acceptable carrier.

11. The use of a compound in accordance with any one of claims 1 to 9 for preparing a pharmaceutical composition useful for inhibiting the action of enkephalinase.

12. The use of a compound in accordance with any one of claims 1 to 9 for preparing a pharmaceutical composition for oral administration to inhibit the action of enkephalinase.

**Claims for the Contracting State: AT**

1. A method of preparing a compound having the formula I

$$R_1C^*(HCOR_2)—NH—C^*(HR_3)—CONH(CH_2)_p—C^*(R_4R_5)—COR_6$$

or a mixture of enantiomers or diasterioisomers containing the same, and the pharmaceutically acceptable salts thereof wherein:

$R_1$ is alkyl having from 1 to 6 carbon atoms, adamantylmethyl, cycloalkylmethyl having from 4 to 8 carbon atoms or $A—X_m—C_nH_{2n}—$ wherein X is oxygen or sulfur, A is phenyl which may be substituted with the group Y, wherein Y is halogen, hydroxy, trifluoromethyl; alkoxy having from 1 to 6 carbon atoms, alkyl having from 1 to 6 carbon atoms, 2- or 3-furanyl, 2- or 3-thienyl, or phenyl (which may be substituted with halogen, hydroxy, trifluoromethyl, alkoxy having from 1 to 6 carbon atoms or alkyl having from 1 to 6

carbon atoms, or may also be benzyl (the phenyl ring of which may be substituted with the group Y, as defined above), 1- or 2-naphthyl, 2- or 3-furanyl or 2- or 3-thienyl; m is 0 or 1 and n is 0, 1, 2, 3, or 4;

$R_2$ and $R_6$ may be the same or different and are hydroxy, alkoxy having from 1 to 8 carbon atoms, B—$X_m$—$C_nH_{2n}$—O— wherein B is phenyl (which may be substituted with the group Y, as defined herein) or 1- or 2-naphthyl, X, m, and n are as defined herein provided that when n = 0, m = 0, —$OCH_2OCO$-alkyl in which the alkyl group has 1 to 6 carbon atoms, —$OCH_2CO$-phenyl (the phenyl ring of which may be substituted with the group Y, as defined above), 1-glyceryl,

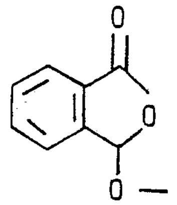

or

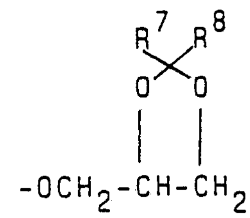

wherein $R_7$ is hydrogen, alkyl having from 1 to 6 carbon atoms, or phenyl which may be substituted with the group Y, as defined above, and $R_8$ is hydrogen or alkyl having from 1 to 6 carbon atoms;

$R_2$ may also be —$NR_7R_8$ wherein $R_7$ and $R_8$ are as defined above;

$R_3$ is alkyl having from 1 to 6 carbon atoms, cycloalkylmethyl having from 4 to 8 carbon atoms, 2- or 3-thienylmethyl, 2- or 3-furanylmethyl, 1- or 2-naphthylmethyl, or benzyl the phenyl ring of which may be substituted with the group Y, as defined above;

$R_4$ is D—$C_nH_{2n}$—$O_m$— wherein D is hydrogen, alkyl having from 1 to 4 carbon atoms or phenyl which may be substituted with the group Z, wherein Z is halogen, hydroxy, trifluoromethyl, alkoxy having from 1 to 6 carbon atoms, or alkyl having from 1 to 6 carbon atoms;

m and n are as defined above;

$R_5$ is hydrogen or alkyl having from 1 to 6 carbon atoms; and

p is 0, 1 or 2;

subject to the following provisos

(i) that when $R_4$ and $R_5$ are both hydrogen, and p is 1 or 2 then;

a) $R_1$ is chosen from adamantyl, 4-phenylphenylethyl, A—$X_m$—$C_nH_{2n}$— wherein m is 1, X is as defined above and *n* is 1, 2, 3, or 4, 1- or 2-naphthyl, 2- or 3-furanyl and 2- or 3-thienyl; and/or

b) $R_2$ and/or $R_6$ are chosen from B—Xm—$C_nH_{2n}$—O— wherein B is as defined above, m is one, X is as defined above and n is 1, 2, 3 or 4, —$OCH_2OCO$-alkyl in which the alkyl group has 1 to 6 carbon atoms, —$OCH_2CO$-phenyl (in which the phenyl group may be substituted by the group Y, as defined herein), 1-glyceryl,

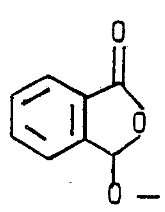

or

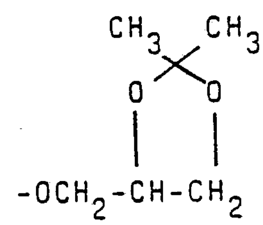

and $R_2$ may also be —$NR_7R_8$ wherein $R_7$ and $R_8$ are as defined herein; and/or

c) $R_3$ is 2- or 3-furanylmethyl or 4-phenylphenylethyl;

(ii) when P is zero then

$R_1$ is benzyl or benzylthiomethyl;

$R_3$ is benzyl or maybe leucyl when $R_1$ is benzyl;

$R_4$ is benzyl when $R_1$ is benzyl, and is methyl when $R_1$ is benzylthiomethyl;

$R_5$ is hydrogen;

characterized in that the compound is prepared by an appropriate method selected from the following methods (wherein in the following formulae p, $R_1$, $R_2$, $R_3$, $R_4$, $R_5$, and $R_6$ are as defined above including suitable protection of any reactive groups);

a) reducing a compound of formula XX at the C = N double bond

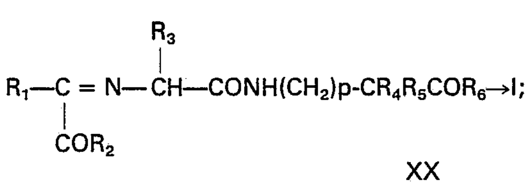

XX

b) condensing a keto acid or ester of formula II with an amino acid of formula III in a reactive medium containing a reducing agent

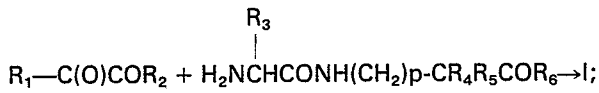

II III

c) coupling an amino acid of formula VIII with an amino acid of formula V

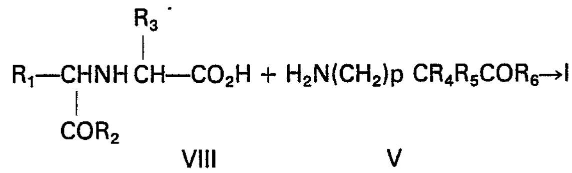

VIII V

d) reducing a compound of the formula XXI at the C = N double bond

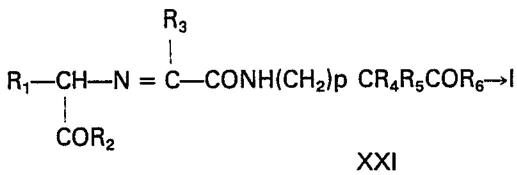

XXI

e) condensing a keto acid or ester of formula X with an amino acid in a reactive medium containing a reducing agent

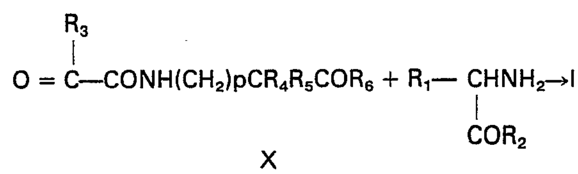

X

f) alkylating an amine of formula III with a compound of formula XXII

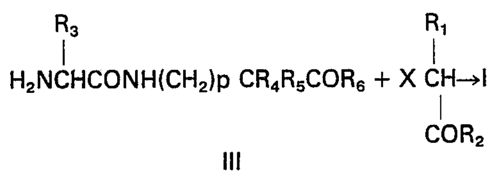

III XXII

in which X is a halogen atom;

followed by removal of any protecting groups if necessary to obtain the desired compound of formula I, and thereafter, if desired converting the compound of formula I into another compound of formula I and/or forming a salt thereof, and if desired isolating a preferred isomer.

2. A method as defined in claim I, characterized in that a compound of formula I is produced in which $R_4$ represents the group $D—C_nH_{2n}—O_m—$ as defined in claim 1 in which the sum of n and m is at least one.

3. A method as defined in claim 1 or 2, characterized in that a compound of formula I is produced in which one of $R_2$ and $R_6$ is 2-phenoxyethyoxy, 1-glyceryl,

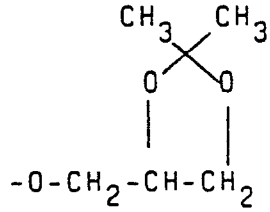 , 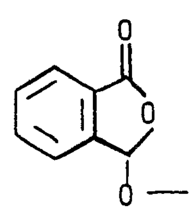 ,

or (2,2-dimethyl-1-oxopropoxy)methoxy, and the other is chosen from the groups defined above or is hydroxy, methoxy, ethoxy or benzyloxy.

4. A method as defined in claim 1 or 2, characterized in that a compound is produced in which $R_1$ is benzyl optionally *para* substituted by chlorine, methoxy, methyl, or phenyl, 2-phenylethyl or 1- or 2-naphthylmethyl,

$R_2$ and $R_6$ are the same or different and are hydroxy, methoxy, ethoxy, benzyloxy, 2-phenoxyethoxy, 1-glyceryl,

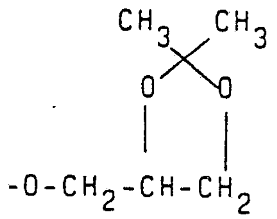 , 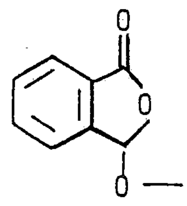 ,

or (2,2-dimethyl-1-oxypropoxy)methoxy, and

$R_3$ is benzyl, p-methylbenzyl, p-phenylbenzyl, 1-naphthylmethyl or 3-thienylmethyl.

5. A method as defined in claim 4, characterized in that a compound is produced in which $R_4$ is methyl or benzyl and $R_5$ is hydrogen.

6. A method as defined in claim 3, characterized in that a compound is produced in which $R_1$ and $R_3$ are as defined in claim 4, $R_4$ is hydrogen, methyl or benzyl, and $R_5$ is hydrogen.

7. A method as defined in claim 6, characterized in that $R_4$ is hydrogen.

8. A method as defined in any one of the preceding claims, characterized in that a compound is produced in which p is one.

9. A method as defined in claim 1, characterized in that the compound

N-[N-[L-1-(2,2-dimethyl-1-oxopropoxy)methoxy]carbonyl]-2-phenylethyl]-L-phenylalanyl]-ß-alanine, (2,2-dimethyl-1-oxopropoxy)methyl ester;

N-[N-[L-1-(phenylmethoxy)carbonyl]-2-phenylethyl]-L-phenylalanyl]-β-alanine, (2,2-dimethyl-1-oxopropoxy)methyl ester;

N-[N-[L-1-carboxy-2-(4-phenyl)phenylethyl]-L-phenylalanyl]-β-alanine;

N-[N-[L-[1-[(2,2-dimethyl-1-oxopropoxy)methoxy]carbonyl]-2-(4-phenyl)phenylethyl]-L-phenylalanyl)-β-alanine, (2,2-dimethyl-1-oxopropoxy)methyl ester;

N-[N-[L-1-carboxy-2-phenylethyl]-L-(4-phenyl)phenylalanyl]-β-alanine;

N-[N-[L-[1-[(2,2-dimethyl-1-oxopropoxy)methoxy]carbonyl]-2-(4-phenyl)phenylethyl]-L-(4-phenyl)phenylalanyl-β-alanine, (2,2-dimethyl-1-oxopropoxy)methyl ester;

N-[N-[L-[1-[(2,2-dimethyl-1-oxopropoxy)methoxy]carbonyl]-2-phenylethyl]-L-(4-phenyl)phenylalanyl]-β-alanine, (2,2-dimethyl-1-oxopropoxy)methyl ester;

N-[N-[L-1-carboxy-2-phenylethyl]-L-phenylalanyl]-L-(α-methyl)-β-alanine;

N-[N-[L-[1-[(2,2-dimethyl-1-oxopropoxy)methoxy]carbonyl]-2-phenylethyl]-L-phenylalanyl]-L-(α-methyl)-β-alanine, (2,2-dimethyl-1-oxopropoxy)methyl ester;

N-[N-[(L-1-carboxy-2-phenylethyl)]-L-phenylalanyl]-β-alanine, 2-phenoxyethyl ester;

N-[N-[L-[1-[(2,2-dimethyl-1-oxopropoxy)methoxy]carbonyl]-2-phenylethyl]-L-phenylalanyl]-β-alanine;

N-[N-[L-[1-[(2-phenoxy)ethoxy]carbonyl]-2-phenylethyl]-L-phenylalanyl]-β-alanine;

N-[N-[L-[1-[(1-oxo-3-isobenzofuranyloxy)]carbonyl]-2-phenylethyl]-L-phenylalanyl-β-alanine;

N-[N-[L-[1-[(2,3-dihyroxy-1-propoxy]carbonyl]-2-phenylethyl]-L-phenylalanyl]-β-alanine;

N-[N-[L-[1-[(2,2-dimethyl-1,3-dioxolan-4-yl)methoxy]carbonyl]-2-phenylethyl]-L-phenylananyl]-β-alanine;

N-[N-[L-[1-[(2,2-dimethyl-1-oxopropoxy)methoxy]carbonyl]-2-(4-phenyl)phenylethyl]-L-phenylalanyl]-β-alanine;

N-[N-[L-[1-[(2-phenoxy)ethoxy]carbonyl]-2-(4-phenyl)phenylethyl]-L-phenylalanyl]-β-alanine;

N-[N-[L-[1-[(1-oxo-3-isobenzofuranyloxy)]carbonyl]-2-(4-phenyl)phenylethyl]-L-phenylalanyl]-β-alanine;

N-[N-[L-[1-[(2,3-dihydroxy)-1-propoxy]carbonyl]-2-(4-phenyl)phenylethyl]-L-phenylalanyl]-β-alanine;

N-[N-[L-[1-[(2,2-dimethyl-1,3-dioxolan-4-yl)methoxy]carbonyl]-2-(4-phenyl)phenylethyl]-L-phenylalanyl]-β-alanine;

N-[N-[L-[1-[(2,2-dimethyl-1-oxopropoxy)methoxy]carbonyl]-2-phenylethyl]-L-(4-phenyl)phenylalanyl]-β-alanine;

N-[N-[L-[1-[(2-phenoxy)ethoxy]carbonyl]-2-phenylethyl]-L-(4-phenyl)phenylalanyl]-β-alanine;

N-[N-[L-[1-[(1-oxo-3-isobenzofuranyloxy)]carbonyl]-2-phenylethyl]-L-(4-phenyl)phenylalanyl]-β-alanine;

N-[N-[L-[1-[(2,3-dihydroxy)-1-propoxy]carbonyl]-2-phenylethyl]-L-(4-phenyl)phenylalanyl]-β-alanine;

N-[N-[L-[1-[(2,2-dimethyl-1,3-dioxolan-4-yl)methoxy]carbonyl]-2-phenylethyl]-L-(4-phenyl)phenylalanyl]-β-alanine;

N-[N-[L-[1-[(2,2-dimethyl-1-oxopropoxy)methoxy]carbonyl]-2-(4-phenyl)phenylethyl]-L-(4-phenyl)phenylalanyl]-β-alanine;

N-[N-[L-[1-[(2-phenoxy)ethoxy]carbonyl]-2-(4-phenyl)phenylethyl]-L-(4-phenyl)phenylalanyl]-β-alanine;

N-[N-[L-[1-[(1-oxo-3-isobenzofuranyloxy)]carbonyl]-2-(4-phenyl)phenylethyl]-L-(4-phenyl)phenylalanyl]-β-alanine;

N-[N-[L-[1-[(2,3-dihydroxy)-1-propoxy]carbonyl]-2-(4-phenyl)phenylethyl]-L-(4-phenyl)phenylalanyl]-β-alanine;

N-[N-[L-[1-[(2,2-dimethyl-1,3-dioxolan-4-yl)methoxy]carbonyl]-2-(4-phenyl)phenylethyl]-L-(4-phenyl)phenylalanyl]-β-alanine;

N-[N-[L-[1-[(2,2-dimethyl-1-oxopropoxy)methoxy]carbonyl]-2-phenylethyl]-L-phenylalanyl]-D,L-α-methyl-β-alanine;

N-[N-[L-[1-[(2-phenoxy)ethoxy]carbonyl]-2-phenylethyl]-L-phenylalanyl]-D,L-α-methyl-β-alanine;

N-[N-[L-[1-[(1-oxo-3-isobenzofuranyloxy)]carbonyl]-2-phenyethyl]-L-phenylalanyl]-D,L-α-methyl-β-alanine;

N-[N-[L-[1-[(2,3-dihydroxy)-1-propoxy]carbonyl]-2-phenylethyl]-L-phenylalanyl]-D,L-α-methyl-β-alanine;

N-[N-[L-[1-[(2,2-dimethyl-1,3-dioxolan-4-yl)methoxy]carbonyl]-2-phenylethyl]-L-phenylalanyl]-D,L-α-methyl-β-alanine;

N-[N-[L-[1-[(2,2-dimethyl-1-oxopropoxy)methoxy]carbonyl]-2-(4-phenyl)phenylethyl]-L-phenylalanyl]-D,L-α-methyl-β-alanine;

N-[N-[L-[1-[(2-phenoxy)ethoxy]carbonyl]-2-(4-phenyl)phenylethyl]-L-phenylalanyl]-D,L-α-methyl-β-alanine;

N-[N-[L-[1-[(1-oxo-3-isobenzofuranyloxy)]carbonyl]-2-(4-phenyl)phenylethyl]-L-phenylalanyl]-D,L-α-methyl-β-alanine;

N-[N-[L-[1-[(2,3-dihydroxy)-1-propoxy]carbonyl]-2-(4-phenyl)phenylethyl]-L-phenylalanyl]-D,L-α-methyl-β-alanine;

N-[N-[L-[1-[(2,2-dimethyl-1,3-dioxolan-4-yl)methoxy]carbonyl]-2-(4-phenyl)phenylethyl]-L-phenylalanyl]-D,L-α-methyl-β-alanine;

N-[N-[L-[1-[(2,2-dimethyl-1-oxopropoxy)methoxy]carbonyl]-2-phenylethyl]-L-(4-phenyl)phenylalanyl]-D,L-α-methyl-β-alanine;

N-[N-[L-[1-[(2-phenoxy)ethoxy]carbonyl]-2-phenylethyl]-L-(4-phenyl)phenylalanyl]-D,L-α-methyl-β-alanine;

N-[N-[L-[1-[(1-oxo-3-isobenzofuranyloxy)]carbonyl]-2-phenylethyl]-L-(4-phenyl)phenylalanyl]-D,L-α-methyl-β-alanine;

N-[N-[L-[1-[(2,3-dihydroxy)-1-propoxy]carbonyl]-2-phenylethyl]-L-(4-phenyl)phenylalanyl]-D,L-α-methyl-β-alanine;

N-[N-[L-[1-[(2,2-dimethyl-1,3-dioxolan-4-yl)methoxy]carbonyl]-2-phenylethyl]-L-(4-phenyl)phenylalanyl]-D,L-α-methyl-β-alanine;

N-[N-[L-[1-[(2,2-dimethyl-1-oxopropoxy)methoxy]carbonyl]-2-(4-phenyl)phenylethyl]-L-(4-phenyl)phenylalanyl]-D,L-α-methyl-β-alanine;

N-[N-[L-[1-[(2-phenoxy)ethoxy]carbonyl]-2-(4-phenyl)phenylethyl]-L-(4-phenyl)phenylalanyl]-D,L-α-methyl-β-alanine;

N-[N-[L-[1-[(1-oxo-3-isobenzofuranyloxy)]carbonyl]-2-(4-phenyl)phenylethyl]-L-(4-phenyl)phenylalanyl]-D,L-α-methyl-β-alanine;

N-[N-[L-[1-[(2,3-dihydroxy)-1-propoxy]carbonyl]-2-(4-phenyl)phenylethyl]-L-(4-phenyl)phenylalanyl]-D,L-α-methyl-β-alanine;

N-[N-[L-[1-[(2,2-dimethyl-1,3-dioxolan-4-yl)methoxy]carbonyl]-2-(4-phenyl)phenylethyl]-L-(4-phenyl)phenylalanyl]-D,L-α-methyl-β-alanine;

N-[N-[L-[1-[(2,2-dimethyl-1-oxopropoxy)methoxy]carbonyl]-2-phenylethyl]-L-2-thienylalanyl]-β-alanine;

N-[N-[L-[1-[(2,2-dimethyl-1-oxopropoxy)methoxy]carbonyl]-2-phenylethyl]-L-3-thienylalanyl]-β-alanine;

N-[N-[L-[1-[(2,2-dimethyl-1-oxopropoxy)methoxy]carbonyl]-2-phenylethyl]-L-2-furoalanyl]-β-alanine;

N-[N-[L-[1-[(2,2-dimethyl-1-oxopropoxy)methoxy]carbonyl]-2-phenylethyl]-L-phenylalanyl]-α-methylalanine;

N-[N-[L-[1-[(2,2-dimethyl-1-oxopropoxy)methoxy]carbonyl]-2-phenylethyl]-L-phenylalanyl]-L-α-hydroxy-β-alanine;

N-[N-[L-[1-[(2,2-dimethyl-1-oxopropoxy)methoxy]carbonyl]-2-phenylethyl]-L-phenylalanyl]-D-α-hydroxy-β-alanine;

N-[N-[L-[1-[(2,2-dimethyl-1-oxopropoxy)methoxy]carbonyl]-2-phenylethyl]-L-phenylalanyl]-L-α-methoxy-β-alanine;

N-[N-[L-[1-[(2,2-dimethyl-1-oxopropoxy)methoxy]carbonyl]-2-phenylethyl]-L-phenylalanyl]-D-α-methoxy-β-alanine;

N-[N-[L-[1-[(2,2-dimethyl-1-oxopropoxy)methoxy]carbonyl]-2-phenylethyl]-L-phenylalanyl]-D,L-α-methyl-β-alanine benzyl ester;

N-[N-[L-[1-(2,2-dimethyl-1-oxopropoxy)methoxy]carbonyl]-2-(4-methoxy)phenylethyl]-L-phenylalanyl-β-alanine;

N-[(L)-1-carboxy-2-phenylethyl]-L-phenyl alanyl-L-phenylalanine;

N-[(L)-1-carboxy-2-phenylethyl]-L-leucyl-L-phenylalanine; or a pharmaceutically acceptable salt is produced.

10. A method of making a pharmaceutical composition, characterized in that a compound of formula I as defined in claim 1 or a mixture of enantiomers or diastereoisomers containing the same or a pharmaceutically acceptable salt thereof, is mixed with a pharmaceutically acceptable carrier or excipient.

11. A method of making a pharmaceutical composition characterized in that a compound of formula I as defined in claim 1 or a mixture of enantiomers or diastereoisomers containing the same, or a pharmaceutically acceptable salt thereof, prepared by the process of any one of the preceding claims, is mixed with a pharmaceutically acceptable carrier or excipient.

12. The use of a compound produced in accordance with any one of claims 1 to 9 for preparing a pharmaceutical composition useful for inhibiting the action of enkephalinase.

13. The use of a compound produced in accordance with any one of claims 1 to 9 for preparing a pharmaceutical composition for oral administration to inhibit the action of enkephalinase.

**Patentansprüche für die Vertragsstaaten: BE CH DE FR GB IT LI LU NL SE**

1. Verbindung der Formel I

$$R_1C^*(HCOR_2)—NH—C^*(HR_3)—CONH(CH_2)_p—C^*(R_4R_5)—COR_6$$

oder ein diese enthaltendes Enantiomeren- oder Diastereomeren-Gemisch und deren pharmazeutisch annehmbare Salze, worin

$R_1$ Alkyl mit 1 bis 6 Kohlenstoff-Atomen, Adamantylmethyl, Cycloalkylmethyl mit 4 bis 8 Kohlenstoff-Atomen oder $A—X_m—C_nH_{2n}—$ ist, worin X Sauerstoff oder Schwefel ist, A Phenyl ist, das mit der Gruppe Y substituiert sein kann, wobei Y Halogen, Hydroxy, Trifluoromethyl, Alkoxy mit 1 bis 6 Kohlenstoff-Atomen, Alkyl mit 1 bis 6 Kohlenstoff-Atomen, 2- oder 3-Furanyl, 2- oder 3-Thienyl oder Phenyl ist [das mit Halogen, Hydroxy, Trifluoromethyl, Alkoxy mit 1 bis 6 Kohlenstoff-Atomen oder Alkyl mit 1 bis 6 Kohlenstoff-Atomen substituiert sein kann] oder auch Benzyl, [dessen Phenyl-Ring mit der oben definierten Gruppe Y substituiert sein kann], 1- oder 2-Naphthyl, 2- oder 3-Furanyl oder 2- oder 3-Thienyl sein kann; m 0 oder 1 ist un n 0, 1, 2, 3 oder 4 ist;

$R_2$ und $R_6$ gleich oder verschieden sein können und Hydroxy, Alkoxy mit 1 bis 8 Kohlenstoff-Atomen, $B—X_m—C_nH_{2n}—O—$, worin B Phenyl [das mit der oben definierten Gruppe Y substituiert sein kann] oder 1- oder 2-Naphthyl ist, X, m und n die angegebenen Bedeutungen haben, mit der Maßgabe, daß m = 0, wenn n = 0, $—OCH_2OCO$-Alkyl, worin die Alkyl-Gruppe 1 bis 6 Kohlenstoff-Atome hat, $—OCH_2CO$-Phenyl [dessen Phenyl-Ring mit der oben definierten Gruppe Y substituiert sein kann], 1-Glyceryl,

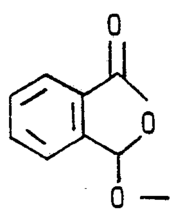

oder

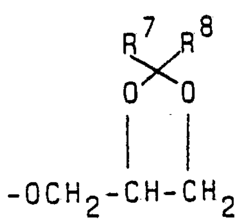

worin $R_7$ Wasserstoff, Alkyl mit 1 bis 6 Kohlenstoff-Atomen, oder Phenyl ist, das mit der oben definierten Gruppe Y substituiert sein kann, und $R_8$ Wasserstoff oder Alkyl mit 1 bis 6 Kohlenstoff-Atomen ist, sind;

$R_2$ auch $—NR_7R_8$ sein kann, worin $R_7$ und $R_8$ die oben angegebenen Bedeutungen haben;

$R_3$ Alkyl mit 1 bis 6 Kohlenstoff-Atomen, Cycloalkylmethyl mit 4 bis 8 Kohlenstoff-Atomen, 2- oder 3-Thienylmethyl, 2- oder 3-Furanylmethyl, 1- oder 2-Naphthylmethyl oder Benzyl ist, dessen Phenyl-Ring mit der oben definierten Gruppe Y substituiert sein kann

$R_4$ $D—C_nH_{2n}—O_m—$ ist, worin D Wassertoff, Alkyl mit 1 bis 4 Kohlenstoff-Atomen oder Phenyl ist, das durch die Gruppe Z substituiert sein kann, wobei Z Halogen, Hydroxy, Trifluoromethyl, Alkoxy mit 1 bis 6 Kohlenstoff-Atomen oder Alkyl mit 1 bis 6 Kohlenstoff-Atomen ist und m und n die oben angegebenen Bedeutungen haben;

$R_5$ Wasserstoff oder Alkyl mit 1 bis 6 Kohlenstoff-Atomen ist und p 0, 1 oder 2 ist;

mit den folgenden Maßgaben,

(i) daß, wenn sowohl $R_4$ als auch $R_5$ Wasserstoff sind und p 1 oder 2 ist, dann

a) $R_1$ aus Adamantyl, 4-Phenylphenylethyl, $A—X_m—C_nH_{2n}—$, worin m 1 ist, X die oben angegebenen Bedeutungen hat und n 1, 2, 3 oder 4 ist, 1- oder 2-Naphthyl, 2- oder 3-Furanyl und 2- oder 3-Thienyl gewählt ist; und/oder

b) $R_2$ und/oder $R_6$ aus $B—X_m—C_nH_{2n}—O—$, worin B die oben angegebenen Bedeutungen hat, m eins ist, X die oben angegebenen Bedeutungen hat und n 1, 2, 3 oder 4 ist, $—OCH_2OCO—$Alkyl, worin die Alkyl-Gruppe 1 bis 6 Kohlenstoff-Atome hat, $—OCH_2CO$-Phenyl [worin die Phenyl-Gruppe mit der oben definierten Gruppe Y substituiert sein kann], 1-Glyceryl,

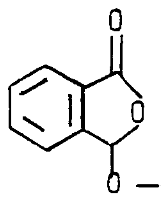

oder

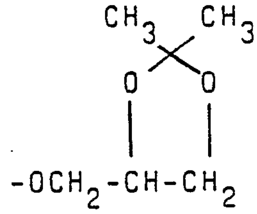

gewählt sind und $R_2$ auch $—NR_7R_8$ sein kann, worin $R_7$ und $R_8$ die oben angegebenen Bedeutungen haben; und/oder

c) $R_3$ 2- oder 3-Furanylmethyl oder 4-Phenylphenylethyl ist;

(ii) daß, wenn p Null ist, dann

$R_1$ Benzyl oder Benzylthiomethyl ist;

$R_3$ Benzyl ist oder Leucyl sein kann, wenn $R_1$ Benzyl ist;

$R_4$ Benzyl ist, wenn $R_1$ Benzyl ist, und Methyl ist, wenn $R_1$ Benzylthiomethyl ist;

$R_5$ Wasserstoff ist.

2. Verbindung der Formel I nach Anspruch 1, dadurch gekennzeichnet, daß $R_4$ eine wie in Anspruch 1 definierte Gruppe $D—C_nH_{2n}—O_m—$ bezeichnet, in der die Summe aus n und m wenigstens eins ist.

3. Verbindung der Formel I nach den Ansprüchen 1 oder 2, dadurch gekennzeichnet, daß einer der Substituenten $R_2$ und $R_6$ 2-Phenoxyethoxy, 1-Glyceryl,

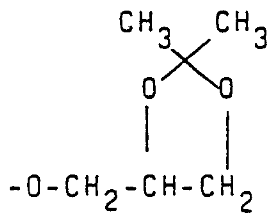

,

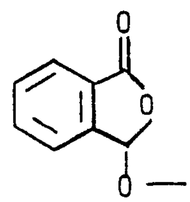

,

oder (2,2-Dimethyl-1-oxopropoxy)methoxy ist und der andere aus den oben definierten Gruppen gewählt oder Hydroxy, Methoxy, Ethoxy oder Benzyloxy ist.

4. Verbindung nach Anspruch 2, dadurch gekennzeichnet, daß

$R_1$ Benzyl, das gegebenenfalls durch Chlor, Methoxy, Methyl oder Phenyl para-substituiert ist, 2-Phenylethyl oder 1- oder 2-Naphthylmethyl ist,

$R_2$ und $R_6$ gleich oder verschieden sind und Hydroxy, Methoxy, Ethoxy, Benzyloxy, 2-Phenoxyethoxy, 1-Glyceryl,

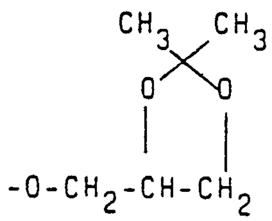

,

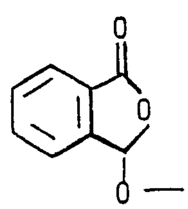

,

oder (2,2-Dimethyl-1-oxopropoxy)methoxy sind und

$R_3$ Benzyl, p-Methylbenzyl, p-Phenylbenzyl, 1-Naphthylmethyl oder 3-Thienylmethyl ist.

5. Verbindung nach Anspruch 4, dadurch gekennzeichnet, daß

$R_4$ Methyl oder Benzyl ist und

$R_5$ Wasserstoff ist.

6. Verbindung nach Anspruch 3, dadurch gekennzeichnet, daß

$R_1$ und $R_3$ die in Anspruch 4 angegebenen Bedeutungen haben,

$R_4$ Wasserstoff, Methyl oder Benzyl ist und

$R_5$ Wasserstoff ist.

7. Verbindung nach Anspruch 6, dadurch gekennzeichnet, daß

$R_4$ Wasserstoff ist.

8. Verbindung nach irgendeinem der vorhergehenden Ansprüche, worin p eins ist.

9. N-[N-[L-[1-[(2,2-Dimethyl-1-oxopropoxy)methoxy]carbonyl]-2-phenylethyl]-L-phenylalanyl-β-alanin-(2,2-dimethyl-1-oxopropoxy)methylester;

N-[N-[L-1-[Phenylmethoxy]carbonyl]-2-phenylethyl]-L-phenylalanyl-β-alanin-(2,2-dimethyl-1-oxopropoxy)methylester;

N-[N-[L-1-Carboxy-2-(4-phenyl)phenylethyl]-L-phenylalanyl-β-alanin

N-[N-[L-[1-[(2,2-Dimethyl-1-oxopropoxy)methoxy]carbonyl]-2-(4-phenyl)phenylethyl]-L-phenylalanyl-β-alanin-(2,2-dimethyl-1-oxopropoxy)methylester;
N-[N-[L-1-Carboxy-2-phenylethyl]-L-(4-phenyl)phenylalanyl-β-alanin;
N-[N-[L-[1-[(2,2-Dimethyl-1-oxopropoxy)methoxy]carbonyl]-2-(4-phenyl)phenylethyl]-L-(4-phenyl)phenylalanyl-β-alanin-(2,2-dimethyl-1-oxopropoxy)methylester;
N-[N-[L-[1-[(2,2-Dimethyl-1-oxopropoxy)methoxy]carbonyl]-2-phenylethyl]-L-(4-phenyl)phenylalanyl-β-alanin-(2,2-dimethyl-1-oxopropoxy)methylester;
N-[N-[L-1-Carboxy-2-phenylethyl]-L-phenylalanyl]-L-(α-methyl)-β-alanin;
N-[N-[L-[1-[(2,2-Dimethyl-1-oxopropoxy)methoxy]carbonyl]-2-phenylethyl]-L-phenylalanyl]-L-(α-methyl)-β-alanin-(2,2-dimethyl-1-oxopropoxy)methylester;
N-[N-[L-1-Carboxy-2-phenylethyl]-L-phenylalanyl]-β-alanin-2-phenoxyethylester;
N-[N-[L-[1-[(2,2-Dimethyl-1-oxopropoxy)methoxy]carbonyl]-2-phenylethyl]-L-phenylalanyl]-β-alanin;
N-[N-[L-[1-[(2-Phenoxy)ethoxy]carbonyl]-2-phenylethyl]-L-phenylalanyl]-β-alanin;
N-[N-[L-[1-[(1-Oxo-3-isobenzofuranyloxy)]carbonyl]-2-phenylethyl]-L-phenylalanyl]-β-alanin;
N-[N-[L-[1-[(2,3-Dihydroxy)-1-propoxy]carbonyl]-2-phenylethyl]-L-phenylalanyl]-β-alanin;
N-[N-[L-[1-[(2,2-Dimethyl-1,3-dioxolan-4-yl)methoxy]carbonyl]-2-phenylethyl]-L-phenylalanyl]-β-alanin;
N-[N-[L-[1-[(2,2-Dimethyl-1-oxopropoxy)methoxy]carbonyl]-2-(4-phenyl)phenylethyl]-L-phenylalanyl]-β-alanin;
N-[N-[L-[1-[(2-Phenoxy)ethoxy]carbonyl]-2-(4-phenyl)phenylethyl]-L-phenylalanyl]-β-alanin;
N-[N-[L-[1-[(1-Oxo-3-isobenzofuranyloxy)carbonyl]-2-(4-phenyl)phenylethyl]-L-phenylalanyl]-β-alanin;
N-[N-[L-[1-[(2,3-Dihydroxy)-1-propoxy]carbonyl]-2-(4-phenyl)phenylethyl]-L-phenylalanyl]-β-alanin;
N-[N-[L-[1-[(2,2-Dimethyl-1,3-dioxolan-4-yl)methoxy]carbonyl]-2-(4-phenyl)phenylethyl]-L-phenylalanyl]-β-alanin;
N-[N-[L-[1-[(2,2-Dimethyl-1-oxopropoxy)methoxy]carbonyl]-2-phenylethyl]-L-(4-phenyl)phenylalanyl-β-alanin;
N-[N-[L-[1-[(2-Phenoxy)ethoxy]carbonyl]-2-phenylethyl]-L-(4-phenyl)phenylalanyl]-β-alanin;
N-[N-[L-[1-[(1-Oxo-3-isobenzofuranyloxy)]carbonyl]-2-phenylethyl]-L-(4-phenyl)phenylalanyl-β-alanin;
N-[N-[L-[1-[(2,3-Dihydroxy)-1-propoxy]carbonyl]-2-phenylethyl]-L-(4-phenyl)phenylalanyl-β-alanin;
N-[N-[L-[1-[(2,2-Dimethyl-1,3-dioxolan-4-yl)methoxy]carbonyl]-2-phenylethyl]-L-(4-phenyl)phenylalanyl]-β-alanin;
N-[N-[L-[1-[(2,2-Dimethyl-1-oxopropoxy)methoxy]carbonyl]-2-(4-phenyl)phenylethyl]-L-(4-phenyl)phenylalanyl-β-alanin;
N-[N-[L-[1-[(2-Phenoxy)ethoxy]carbonyl]-2-(4-phenyl)phenylethyl]-L-(4-phenyl)phenylalanyl]-β-alanin;
N-[N-[L-[1-[(1-Oxo-3-isobenzofuranyloxy)]carbonyl]-2-(4-phenyl)phenylethyl]-L-(4-phenyl)phenylalanyl-β-alanin;
N-[N-[L-[1-[(2,3-Dihydroxy)-1-propoxy]carbonyl]-2-(4-phenyl)phenylethyl]-L-(4-phenyl)phenylalanyl-β-alanin;
N-[N-[L-[1-[(2,2-Dimethyl-1,3-dioxolan-4-yl)methoxy]carbonyl]-2-(4-phenyl)phenylethyl]-L-(4-phenyl)phenylalanyl]-β-alanin;
N-[N-[L-[1-[(2,2-Dimethyl-1-oxopropoxy)methoxy]carbonyl]-2-phenylethyl]-L-phenylalanyl]-D,L-α-methyl-β-alanin;
N-[N-[L-[1-[(2-Phenoxy)ethoxy]carbonyl]-2-phenylethyl]-L-phenylalanyl]-D,L-α-methyl-β-alanin;
N-[N-[L-[1-[(1-Oxo-3-isobenzofuranyloxy)]carbonyl]-2-phenylethyl]-L-phenylalanyl]-D,L-α-methyl-β-alanin;
N-[N-[L-[1-[(2,3-Dihydroxy)-1-propoxy]carbonyl]-2-phenylethyl]-L-phenylalanyl]-D,L-α-methyl-β-alanin;
N-[N-[L-[1-[(2,2-Dimethyl-1,3-dioxolan-4-yl)methoxy]carbonyl]-2-phenylethyl]-L-phenylalanyl]-D,L-α-methyl-β-alanin;
N-[N-[L-[1-[(2,2-Dimethyl-1-oxopropoxy)methoxy]carbonyl]-2-(4-phenyl)phenylethyl]-L-phenylalanyl]-D,L-α-methyl-β-alanin;
N-[N-[L-[1-[(2-Phenoxy)ethoxy]carbonyl]-2-(4-phenyl)phenylethyl]-L-phenylalanyl]-D,L-α-methyl-β-alanin;
N-[N-[L-[1-[(1-Oxo-3-isobenzofuranyloxy)]carbonyl]-2-(4-phenyl)phenylethyl-L-phenylalanyl]-D,L-α-methyl-β-alanin;
N-[N-[L-[1-[(2,3-Dihydroxy)-1-propoxy]carbonyl]-2-(4-phenyl)phenylethyl]-L-phenylalanyl]-D,L-α-methyl-β-alanin;
N-[N-[L-[1-[(2,2-Dimethyl-1,3-dioxolan-4-yl)methoxy]carbonyl]-2-(4-phenyl)phenylethyl]-L-phenylalanyl]-D,L-α-methyl-β-alanin;
N-[N-[L-[1-[(2,2-Dimethyl-1-oxopropoxy)methoxy]carbonyl]-2-phenylethyl]-L-(4-phenyl)phenylalanyl]-D,L-α-methyl-β-alanin;
N-[N-[L-[1-[(2-Phenoxy)ethoxy]carbonyl]-2-phenylethyl]-L-(4-phenyl)phenylalanyl]-D,L-α-methyl-β-alanin;
N-[N-[L-[1-[(1-Oxo-3-isobenzofuranyloxy)]carbonyl]-2-phenylethyl-L-(4-phenyl)phenylalanyl]-D,L-α-methyl-β-alanin;

N-[N-[L-[1-[(2,3-Dihydroxy)-1-propoxy]carbonyl]-2-phenylethyl]-L-(4-phenyl)phenylalanyl]-D,L-α-methyl-β-alanin;

N-[N-[L-[1-[(2,2-Dimethyl-1,3-dioxolan-4-yl)methoxy]carbonyl]-2-(4-phenyl)phenylethyl]-L-(4-phenyl)phenylalanyl]-D,L-α-methyl-β-alanin;

N-[N-[L-[1-[(2,2-Dimethyl-1-oxopropoxy)methoxy]carbonyl]-2-(4-phenyl)phenylethyl]-L-(4-phenyl)phenylalanyl-D,L-α-methyl-β-alanin;

N-[N-[L-[1-[(2-Phenoxy)ethoxy]carbonyl]-2-(4-phenyl)phenylethyl]-L-(4-phenyl)phenylalanyl]-D,L-α-methyl-β-alanin;

N-[N-[L-[1-[(1-Oxo-3-isobenzofuranyloxy)]carbonyl]-2-(4-phenyl)phenylethyl-L-(4-phenyl)phenylalanyl]-D,L-α-methyl-β-alanin;

N-[N-[L-[1-[(2,3-Dihydroxy)-1-propoxy]carbonyl]-2-(4-phenyl)phenylethyl]-L-(4-phenyl)phenylalanyl]-D,L-α-methyl-β-alanin;

N-[N-[L-[1-[(2,2-Dimethyl-1,3-dioxolan-4-yl)methoxy]carbonyl]-2-(4-phenyl)phenylethyl]-L-(4-phenyl)phenylalanyl]-D,L-α-methyl-β-alanin;

N-[N-[L-[1-[(2,2-Dimethyl-1-oxopropoxy)methoxy]carbonyl]-2-phenylethyl]-L-2-thienylalanyl]-β-alanin;

N-[N-[L-[1-[(2,2-Dimethyl-1-oxopropoxy)methoxy]carbonyl]-2-phenylethyl]-L-3-thienylalanyl]-β-alanin;

N-[N-[L-[1-[(2,2-Dimethyl-1-oxopropoxy)methoxy]carbonyl]-2-phenylethyl]-L-2-furoalanyl]-β-alanin;

N-[N-[L-[1-[(2,2-Dimethyl-1-oxopropoxy)methoxy]carbonyl]-2-phenylethyl]-L-phenylalanyl]-α-methylalanin;

N-[N-[L-[1-[(2,2-Dimethyl-1-oxopropoxy)methoxy]carbonyl]-2-phenylethyl]-L-phenylalanyl]-L-α-hydroxy-β-alanin;

N-[N-[L-[1-[(2,2-Dimethyl-1-oxopropoxy)methoxy]carbonyl]-2-phenylethyl]-L-phenylalanyl]-D-α-hydroxy-β-alanin;

N-[N-[L-[1-[(2,2-Dimethyl-1-oxopropoxy)methoxy]carbonyl]-2-phenylethyl]-L-phenylalanyl]-L-α-methoxy-β-alanin;

N-[N-[L-[1-[(2,2-Dimethyl-1-oxopropoxy)methoxy]carbonyl]-2-phenylethyl]-L-phenylalanyl]-D-α-methoxy-β-alanin;

N-[N-[L-[1-[(2,2-Dimethyl-1-oxopropoxy)methoxy]carbonyl]-2-phenylethyl]-L-phenylalanyl]-α-DL-methyl-β-alanin-benzylester;

N-[N-[L-[1-[(2,2-Dimethyl-1-oxopropoxy)methoxy]carbonyl]-2-(4-methoxy)phenylethyl]-L-phenylalanyl]-β-alanin;

N-[(L)-1-Carboxy-2-phenylethyl]-L-phenylalanyl]-L-phenylalanin;

N-[(L)-1-Carboxy-2-phenylethyl]-L-leucyl-L-phenylalanin;

und/oder ein Salz einer der vorhergehenden Verbindungen, die eine freie Säure-Funktion enthält, mit einer pharmazeutisch annehmbaren Base, oder ein Salz einer der vorhergehenden Verbindungen, die eine freie Säure-Funktion enthält, mit einer pharmazeutisch annehmbaren Säure.

10. Zusammensetzung, umfassend eine Verbindung nach irgendeinem der vorhergehenden Ansprüche in Verbindung mit einem pharmazeutisch annehmbaren Träger.

11. Verwendung einer Verbindung nach irgendeinem der Ansprüche 1 bis 9 zur Herstellung einer pharmazeutischen Zusammensetzung, die zur Hemmung der Enkephalinase-Wirkung von Nutzen ist.

12. Verwendung einer Verbindung nach irgendeinem der Ansprüche 1 bis 9 zur Herstellung einer pharmazeutischen Zusammensetzung für die orale Verabreichung zur Hemmung der Enkephalinase-Wirkung.

**Patentansprüche für die Vertragsstaat: AT**

1. Verfahren zur Herstellung einer Verbindung der Formel I

$$R_1C^*(HCOR_2)—NH—C^*(HR_3)—CONH(CH_2)_p—C^*(R_4R_5)—COR_6$$

oder eines diese enthaltenden Enantiomeren- oder Diastereomeren-Gemischs und der pharmazeutisch annehmbaren Salze derselben, worin

$R_1$ Alkyl mit 1 bis 6 Kohlenstoff-Atomen, Adamantylmethyl, Cycloalkylmethyl mit 4 bis 8 Kohlenstoff-Atomen oder $A—X_m—C_nH_{2n}—$ ist, worin X Sauerstoff oder Schwefel ist, A Phenyl ist, das mit der Gruppe Y substituiert sein kann, wobei Y Halogen, Hydroxy, Trifluoromethyl, Alkoxy mit 1 bis 6 Kohlenstoff-Atomen, Alkyl mit 1 bis 6 Kohlenstoff-Atomen, 2- oder 3-Furanyl, 2- oder 3-Thienyl oder Phenyl ist [das mit Halogen, Hydroxy, Trifluoromethyl, Alkoxy mit 1 bis 6 Kohlenstoff-Atomen oder Alkyl mit 1 bis 6 Kohlenstoff-Atomen substituiert sein kann] oder auch Benzyl (dessen Phenyl-Ring mit der oben definierten Gruppe Y substituiert sein kann], 1- oder 2-Naphthyl, 2- oder 3-Furanyl oder 2- oder 3-Thienyl sein kann; m 0 oder 1 ist un n 0, 1, 2, 3 oder 4 ist;

$R_2$ und $R_6$ gleich oder verschieden sein können und Hydroxy, Alkoxy mit 1 bis 8 Kohlenstoff-Atomen, $B—X_m—C_nH_{2n}—O—$, worin B Phenyl [das mit der oben definierten Gruppe Y substituiert sein kann] oder 1- oder 2-Naphthyl ist, X, m und n die angegebenen Bedeutungen haben, mit der Maßgabe, daß m = 0, wenn

n = 0, —$OCH_2OCO$-Alkyl, worin die Alkyl-Gruppe 1 bis 6 Kohlenstoff-Atome hat, —$OCH_2CO$-Phenyl [dessen Phenyl-Ring mit der oben definierten Gruppe Y substituiert sein kann], 1-Glyceryl,

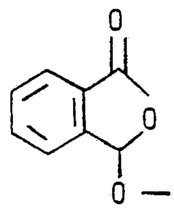

oder

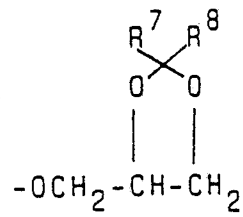

worin $R_7$ Wasserstoff, Alkyl mit 1 bis 6 Kohlenstoff-Atomen, oder Phenyl ist, das mit der oben definierten Gruppe Y substituiert sein kann, und $R_8$ Wasserstoff oder Alkyl mit 1 bis 6 Kohlenstoff-Atomen ist, sind;

$R_2$ auch —$NR_7R_8$ sein kann, worin $R_7$ und $R_8$ die oben angegebenen Bedeutungen haben;

$R_3$ Alkyl mit 1 bis 6 Kohlenstoff-Atomen, Cycloalkylmethyl mit 4 bis 8 Kohlenstoff-Atomen, 2- oder 3-Thienylmethyl, 2- oder 3-Furanylmethyl, 1- oder 2-Naphthylmethyl oder Benzyl ist, dessen Phenyl-Ring mit der oben definierten Gruppe Y substituiert sein kann

$R_4$ D—$C_nH_{2n}$—$O_m$— ist, worin D Wassertoff, Alkyl mit 1 bis 4 Kohlenstoff-Atomen oder Phenyl ist, das durch die Gruppe Z substituiert sein kann, wobei Z Halogen, Hydroxy, Trifluoromethyl, Alkoxy mit 1 bis 6 Kohlenstoff-Atomen oder Alkyl mit 1 bis 6 Kohlenstoff-Atomen ist und m und n die oben angegebenen Bedeutungen haben;

$R_5$ Wasserstoff oder Alkyl mit 1 bis 6 Kohlenstoff-Atomen ist und p 0, 1 oder 2 ist;
mit den folgenden Maßgaben,

(i) daß, wenn sowohl $R_4$ als auch $R_5$ Wasserstoff sind und p 1 oder 2 ist, dann

a) $R_1$ aus Adamantyl, 4-Phenylphenylethyl, A—$X_m$—$C_n$—$H_{2n}$—, worin m 1 ist, X die oben angegebenen Bedeutungen hat und n 1, 2, 3 oder 4 ist, 1- oder 2-Naphthyl, 2- oder 3-Furanyl und 2- oder 3-Thienyl gewählt ist; und/oder

b) $R_2$ und/oder $R_6$ aus B—$X_m$—$C_nH_{2n}$—O—, worin B die oben angegebenen Bedeutungen hat, m eins ist, X die oben angegebenen Bedeutungen hat und n 1, 2, 3 oder 4 ist, —$OCH_2OCO$—Alkyl, worin die Alkyl-Gruppe 1 bis 6 Kohlenstoff-Atome hat, —$OCH_2CO$-Phenyl [worin die Phenyl-Gruppe mit der oben definierten Gruppe Y substituiert sein kann], 1-Glyceryl,

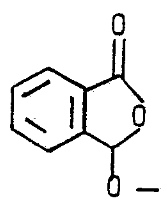

oder

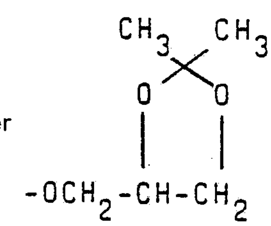

gewählt sind und $R_2$ auch —$NR_7R_8$ sein kann, worin $R_7$ und $R_8$ die oben angegebenen Bedeutungen haben; und/oder

c) $R_3$ 2- oder 3-Furanylmethyl oder 4-Phenylphenylethyl ist;

(ii) daß, wenn p Null ist, dann

$R_1$ Benzyl oder Benzylthiomethyl ist;

$R_3$ Benzyl ist oder Leucyl sein kann, wenn $R_1$ Benzyl ist;

$R_4$ Benzyl ist, wenn $R_1$ Benzyl ist, und Methyl ist, wenn $R_1$ Benzylthiomethyl ist;

$R_5$ Wasserstoff ist.

dadurch gekennzeichnet, daß die Verbindung mittels eines geeigneten Verfahrens hergestellt wird, das aus den folgenden Verfahren ausgewählt ist (wobei in den nachstehenden Formeln p, $R_1$, $R_2$, $R_3$, $R_4$, $R_5$ und $R_6$ die oben angegebenen Bedeutungen haben und geeigneten Schutz etwaiger reaktionsfähiger Gruppen einschließen);

a) Reduzieren einer Verbindung der Formel XX an der C=N -Doppelbindung

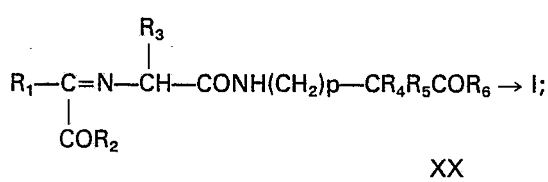

XX

b) Kondensieren einer Ketosäure oder eines Ketoesters der Formel II mit einer Aminosäure der Formel III in einem reaktionsfähigen, ein Reduktionsmittel enthaltenden Medium

$$\begin{array}{c} R_3 \\ | \\ R_1—C(O)COR_2 + H_2NCHCONH(CH_2)p—CR_4R_5COR_6 \rightarrow I; \end{array}$$

II III

c) Kuppeln einer Aminosäure der Formel VIII mit einer Aminosäure der Formel V

$$\begin{array}{c} R_3 \\ | \\ R_1—CHNHCH—CO_2H + H_2N(CH_2)p—CR_4R_5COR_6 \rightarrow I; \\ | \\ COR_2 \end{array}$$

VIII V

d) Reduzieren einer Verbindung der Formel XXI an der C=N -Doppelbindung

$$\begin{array}{c} R_3 \\ | \\ R_1—CH—N=C—CONH(CH_2)p—CR_4R_5COR_6 \rightarrow I; \\ | \\ COR_2 \end{array}$$

XXI

e) Kondensieren einer Ketosäure oder eines Ketoesters der Formel X mit einer Aminosäure in einem reaktionsfähigen, ein Reduktionsmittel enthaltenden Medium

$$\begin{array}{c} R_3 \\ | \\ O=C—CONH(CH_2)p—CR_4R_5COR_6 + R_1—CHNH_2 \rightarrow I; \\ | \\ COR_2 \end{array}$$

X

f) Alkylieren eines Amins der Formel III mit einer Verbindung der Formel XXII

$$\begin{array}{c} R_3 \qquad\qquad R_1 \\ | \qquad\qquad | \\ H_2NCHCONH(CH_2)p—CR_4R_5COR_6 + XCH \rightarrow I; \\ | \\ COR_2 \end{array}$$

III XXII

worin X ein Halogen-Atom ist;
und anschließend erforderlichenfalls zur Gewinnung der Verbindung der Formel I etwaige Schutzgruppen entfernt werden und danach gewünschtenfalls die Verbindung der Formel I in eine andere Verbindung der Formel I überführt und/oder ein Salz derselben gebildet wird und gewünschtenfalls ein bevorzugtes Isomer isoliert wird.

2. Verfahren nach Anspruch 1, dadurch gekennzeichnet, daß eine Verbindung der Formel I hergestellt wird, in der $R_4$ die wie in Anspruch 1 definierte Gruppe $D—C_nH_{2n}—O_m—$ bezeichnet, in der die Summe aus n und m wenigstens eins ist.

3. Verfahren nach den Ansprüchen 1 oder 2, dadurch gekennzeichnet, daß eine Verbindung der Formel I hergestellt wird, in der einer der Substituenten $R_2$ und $R_6$ 2-Phenoxyethoxy, 1-Glyceryl,

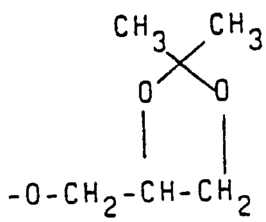,

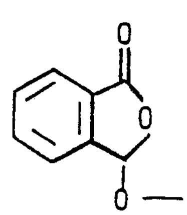,

oder (2,2-Dimethyl-1-oxopropoxy)methoxy ist und der andere aus den oben definierten Gruppen gewählt oder Hydroxy, Methoxy, Ethoxy oder Benzyloxy ist.

4. Verfahren nach den Ansprüchen 1 oder 2, dadurch gekennzeichnet, daß eine Verbindung hergestellt wird, in der

$R_1$ Benzyl, das gegebenenfalls durch Chlor, Methoxy, Methyl oder Phenyl para-substituiert ist, 2-Phenylethyl oder 1- oder 2-Naphthylmethyl ist,

$R_2$ und $R_6$ gleich oder verschieden sind und Hydroxy, Methoxy, Ethoxy, Benzyloxy, 2-Phenoxyethoxy, 1-Glyceryl,

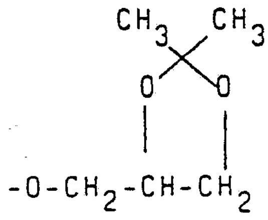 , 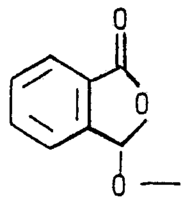 ,

oder (2,2-Dimethyl-1-oxopropoxy)methoxy sind und

$R_3$ Benzyl, p-Methylbenzyl, p-Phenylbenzyl, 1-Napthylmethyl oder 3-Thienylmethyl ist.

5. Verfahren nach Anspruch 4, dadurch gekennzeichnet daß eine Verbindung hergestellt wird, in der
$R_4$ Methyl oder Benzyl ist und
$R_5$ Wasserstoff ist.

6. Verfahren nach Anspruch 3, dadurch gekennzeichnet daß eine Verbindung hergestellt wird, in der
$R_1$ und $R_3$ die in Anspruch 4 angegebenen Bedeutungen haben,
$R_4$ Wasserstoff, Methyl oder Benzyl ist und
$R_5$ Wasserstoff ist.

7. Verfahren nach Anspruch 6, dadurch gekennzeichnet daß eine Verbindung hergestellt wird, in der
$R_4$ Wasserstoff ist.

8. Verfahren nach irgendeinem der vorhergehenden Ansprüche, dadurch gekennzeichnet daß eine Verbindung hergestellt wird, in der p eins ist.

9. Verfahren nach Anspruch 1, dadurch gekennzeichnet, daß die Verbindung

N-[N-[L-[1-[(2,2-Dimethyl-1-oxopropoxy)methoxy]carbonyl]-2-phenylethyl]-L-phenylalanyl-β-alanin-(2,2-dimethyl-1-oxopropoxy)methylester;

N-[N-[L-1-[Phenylmethoxy]carbonyl]-2-phenylethyl]-L-phenylalanyl-β-alanin-(2,2-dimethyl-1-oxopropoxy)methylester;

N-[N-[L-1-Carboxy-2-(4-phenyl)phenylethyl]-L-phenylalanyl-β-alanin;

N-[N-[L-[1-[(2,2-Dimethyl-1-oxopropoxy)methoxy]carbonyl]-2-(4-phenyl)phenylethyl]-L-phenylalanyl-β-alanin-(2,2-dimethyl-1-oxopropoxy)methylester;

N-[N-[L-1-Carboxy-2-phenylethyl]-L-(4-phenyl)phenylalanyl]-β-alanin;

N-[N-[L-[1-[(2,2-Dimethyl-1-oxopropoxy)methoxy]carbonyl]-2-(4-phenyl)phenylethyl]-L-(4-phenyl)phenylalanyl-β-alanin-(2,2-dimethyl-1-oxopropoxy)methylester;

N-[N-[L-[1-[(2,2-Dimethyl-1-oxopropoxy)methoxy]carbonyl]-2-phenylethyl]-L-(4-phenyl)phenylalanyl-β-alanin-(2,2-dimethyl-1-oxopropoxy)methylester;

N-[N-[L-1-Carboxy-2-phenylethyl]-L-phenylalanyl]-L-(α-methyl)-β-alanin;

N-[N-[L-[1-[(2,2-Dimethyl-1-oxopropoxy)methoxy]carbonyl]-2-phenylethyl]-L-phenylalanyl]-L-(α-methyl)-β-alanin-(2,2-dimethyl-1-oxopropoxy)methylester;

N-[N-[L-1-Carboxy-2-phenylethyl]-L-phenylalanyl]-β-alanin-2-phenoxyethylester;

N-[N-[L-[1-[(2,2-Dimethyl-1-oxopropoxy)methoxy]carbonyl]-2-phenylethyl]-L-phenylalanyl]-β-alanin;

N-[N-[L-[1-[(2-Phenoxy)ethoxy]carbonyl]-2-phenylethyl]-L-phenylalanyl]-β-alanin;

N-[N-[L-[1-[(1-Oxo-3-isobenzofuranyloxy)carbonyl]-2-phenylethyl]-L-phenylalanyl]-β-alanin;

N-[N-[L-[1-[(2,3-Dihydroxy)-1-propoxy]carbonyl]-2-phenylethyl]-L-phenylalanyl]-β-alanin;

N-[N-[L-[1-[(2,2-Dimethyl-1,3-dioxolan-4-yl)methoxy]carbonyl]-2-phenylethyl]-L-phenylalanyl]-β-alanin;

N-[N-[L-[1-[(2,2-Dimethyl-1-oxopropoxy)methoxy]carbonyl]-2-(4-phenyl)phenylethyl]-L-phenylalanyl]-β-alanin;

N-[N-[L-[1-[(2-Phenoxy)ethoxy]carbonyl]-2-(4-phenyl)phenylethyl]-L-phenylalanyl]-β-alanin;

N-[N-[L-[1-[(1-Oxo-3-isobenzofuranyloxy)carbonyl]-2-(4-phenyl)phenylethyl]-L-phenylalanyl]-β-alanin;

N-[N-[L-[1-[(2,3-Dihydroxy)-1-propoxy]carbonyl]-2-(4-phenyl)phenylethyl]-L-phenylalanyl]-β-alanin;

N-[N-[L-[1-[(2,2-Dimethyl-1,3-dioxolan-4-yl)methoxy]carbonyl]-2-(4-phenyl)phenylethyl]-L-phenylalanyl]-β-alanin;

N-[N-[L-[1-[(2,2-Dimethyl-1-oxopropoxy)methoxy]carbonyl]-2-phenylethyl]-L-(4-phenyl)phenylalanyl-β-alanin;

N-[N-[L-[1-[(2-Phenoxy)ethoxy]carbonyl]-2-phenylethyl]-L-(4-phenyl)phenylalanyl]-β-alanin;

N-[N-[L-[1-[(1-Oxo-3-isobenzofuranyloxy)]carbonyl]-2-phenylethyl]-L-(4-phenyl)phenylalanyl]-β-alanin;

N-[N-[L-[1-[(2,3-Dihydroxy)-1-propoxy]carbonyl]-2-phenylethyl]-L-(4-phenyl)phenylalanyl]-β-alanin;
N-[N-[L-[1-[(2,2-Dimethyl-1,3-dioxolan-4-yl)methoxy]carbonyl]-2-phenylethyl]-L-(4-phenyl)phenylalanyl]-β-alanin;
N-[N-[L-[1-[(2,2-Dimethyl-1-oxopropoxy)methoxy]carbonyl]-2-(4-phenyl)phenylethyl]-L-(4-phenyl)phenylalanyl-β-alanin;
N-[N-[L-[1-[(2-Phenoxy)ethoxy]carbonyl]-2-(4-phenyl)phenylethyl]-L-(4-phenyl)phenylalanyl]-β-alanin;
N-[N-[L-[1-[(1-Oxo-3-isobenzofuranyloxy)]carbonyl]-2-(4-phenyl)phenylethyl]-L-(4-phenyl)phenylalanyl]-β-alanin;
N-[N-[L-[1-[(2,3-Dihydroxy)-1-propoxy]carbonyl]-2-(4-phenyl)phenylethyl]-L-(4-phenyl)phenylalanyl]-β-alanin;
N-[N-[L-[1-[(2,2-Dimethyl-1,3-dioxolan-4-yl)methoxy]carbonyl]-2-(4-phenyl)phenylethyl]-L-(4-phenyl)phenylalanyl]-β-alanin;
N-[N-[L-[1-[(2,2-Dimethyl-1-oxopropoxy)methoxy]carbonyl]-2-phenylethyl]-L-phenylalanyl]-D,L-α-methyl-β-alanin;
N-[N-[L-[1-[(2-Phenoxy)ethoxy]carbonyl]-2-phenylethyl]-L-phenylalanyl]-D,L-α-methyl-β-alanin;
N-[N-[L-[1-[(1-Oxo-3-isobenzofuranyloxy)]carbonyl]-2-phenylethyl]-L-phenylalanyl]-D,L-α-methyl-β-alanin;
N-[N-[L-[1-[(2,3-Dihydroxy)-1-propoxy]carbonyl]-2-phenylethyl]-L-phenylalanyl]-D,L-α-methyl-β-alanin;
N-[N-[L-[1-[(2,2-Dimethyl-1,3-dioxolan-4-yl)methoxy]carbonyl]-2-phenylethyl]-L-phenylalanyl]-D,L-α-methyl-β-alanin;
N-[N-[L-[1-[(2,2-Dimethyl-1-oxopropoxy)methoxy]carbonyl]-2-(4-phenyl)phenylethyl]-L-phenylalanyl]-D,L-α-methyl-β-alanin;
N-[N-[L-[1-[(2-Phenoxy)ethoxy]carbonyl]-2-(4-phenyl)phenylethyl]-L-phenylalanyl]-D,L-α-methyl-β-alanin;
N-[N-[L-[1-[(1-Oxo-3-isobenzofuranyloxy)]carbonyl]-2-(4-phenyl)phenylethyl-L-phenylalanyl]-D,L-α-methyl-β-alanin;
N-[N-[L-[1-[(2,3-Dihydroxy)-1-propoxy]carbonyl]-2-(4-phenyl)phenylethyl]-L-phenylalanyl]-D,L-α-methyl-β-alanin;
N-[N-[L-[1-[(2,2-Dimethyl-1,3-dioxolan-4-yl)methoxy]carbonyl]-2-(4-phenyl)phenylethyl]-L-phenylalanyl]-D,L-α-methyl-β-alanin;
N-[N-[L-[1-[(2,2-Dimethyl-1-oxopropoxy)methoxy]carbonyl]-2-phenylethyl]-L-(4-phenyl)phenylalanyl]-D,L-α-methyl-β-alanin;
N-[N-[L-[1-[(2-Phenoxy)ethoxy]carbonyl]-2-phenylethyl]-L-(4-phenyl)phenylalanyl]-D,L-α-methyl-β-alanin;
N-[N-[L-[1-[(1-Oxo-isobenzofuranyloxy)]carbonyl]-2-phenylethyl-L-(4-phenyl)phenylalanyl]-D,L-α-methyl-β-alanin;
N-[N-[L-[1-[(2,3-Dihydroxy)-1-propoxy]carbonyl]-2-phenylethyl]-L-(4-phenyl)phenylalanyl]-D,L-α-methyl-β-alanin;
N-[N-[L-[1-[(2,2-Dimethyl-1,3-dioxolan-4-yl)methoxy]carbonyl]-2-(4-phenyl)phenylethyl]-L-(4-phenyl)phenylalanyl]-D,L-α-methyl-β-alanin;
N-[N-[L-[1-[(2,2-Dimethyl-1-oxopropoxy)methoxy]carbonyl]-2-(4-phenyl)phenylethyl]-L-(4-phenyl)phenylalanyl-D,L-α-methyl-β-alanin;
N-[N-[L-[1-[(2-Phenoxy)ethoxy]carbonyl]-2-(4-phenyl)phenylethyl]-L-(4-phenyl)phenylalanyl]-D,L-α-methyl-β-alanin;
N-[N-[L-[1-[(1-Oxo-isobenzofuranyloxy)]carbonyl]-2-(4-phenyl)phenylethyl-L-(4-phenyl)phenylalanyl]-D,L-α-methyl-β-alanin;
N-[N-[L-[1-[(2,3-Dihydroxy)-1-propoxy]carbonyl]-2-(4-phenyl)phenylethyl]-L-(4-phenyl)phenylalanyl]-D,L-α-methyl-β-alanin;
N-[N-[L-[1-[(2,2-Dimethyl-1,3-dioxolan-4-yl)methoxy]carbonyl]-2-(4-phenyl)phenylethyl]-L-(4-phenyl)phenylalanyl]-D,L-α-methyl-β-alanin;
N-[N-[L-[1-[(2,2-Dimethyl-1-oxopropoxy)methoxy]carbonyl]-2-phenylethyl]-L-2-thienylalanyl]-β-alanin;
N-[N-[L-[1-[(2,2-Dimethyl-1-oxopropoxy)methoxy]carbonyl]-2-phenylethyl]-L-3-thienylalanyl]-β-alanin;
N-[N-[L-[1-[(2,2-Dimethyl-1-oxopropoxy)methoxy]carbonyl]-2-phenylethyl]-L-2-furoalanyl]-β-alanin;
N-[N-[L-[1-[(2,2-Dimethyl-1-oxopropoxy)methoxy]carbonyl]-2-phenylethyl]-L-phenylalanyl]-α-methyl-alanin;
N-[N-[L-[1-[(2,2-Dimethyl-1-oxopropoxy)methoxy]carbonyl]-2-phenylethyl]-L-phenylalanyl]-L-α-hydroxy-β-alanin;
N-[N-[L-[1-[(2,2-Dimethyl-1-oxopropoxy)methoxy]carbonyl]-2-phenylethyl]-L-phenylalanyl]-D-α-hydroxy-β-alanin;
N-[N-[L-[1-[(2,2-Dimethyl-1-oxopropoxy)methoxy]carbonyl]-2-phenylethyl]-L-phenylalanyl]-L-α-methoxy-β-alanin;
N-[N-[L-[1-[(2,2-Dimethyl-1-oxopropoxy)methoxy]carbonyl]-2-phenylethyl]-L-phenylalanyl]-D-α-

methoxy-β-alanin;

N-[N-[L-[1-[(2,2-Dimethyl-1-oxopropoxy)methoxy]carbonyl]-2-phenylethyl]-L-phenylalanyl]-α-DL-methyl-β-alanin-benzylester;

N-[N-[L-[1-[(2,2-Dimethyl-1-oxopropoxy)methoxy]carbonyl]-2-(4-methoxy)phenylethyl]-L-phenylalanyl]-β-alanin;

N-[(L)-1-Carboxy-2-phenylethyl]-L-phenylalanyl]-L-phenylalanin;

N-[(L)-1-Carboxy-2-phenylethyl]-L-leucyl-L-phenylalanin;

oder ein pharmazeutisch annehmbares Salz hergestellt wird.

10. Verfahren zur Herstellung einer pharmazeutischen Zusammensetzung, dadurch gekennzeichnet, daß eine Verbindung der Formel I nach Anspruch 1 oder ein diese enthaltendes Enantiomeren- oder Diastereomeren-Gemisch oder ein pharmazeutisch annehmbares Salze derselben mit einem pharmazeutisch annehmbaren Träger oder Streckmittel vermischt wird.

11. Verfahren zur Herstellung einer pharmazeutischen Zusammensetzung, dadurch gekennzeichnet, daß eine Verbindung der Formel I nach Anspruch 1 oder ein diese enthaltendes Enantiomeren- oder Diastereomeren-Gemisch oder ein pharmazeutisch annehmbares Salze derselben, hergestellt nach irgendeinem der vorhergehenden Ansprüche, mit einem pharmazeutisch annehmbaren Träger oder Streckmittel vermischt wird.

12. Verwendung einer nach irgendeinem der Ansprüche 1 bis 9 hergestellten Verbindung zur Herstellung einer pharmazeutischen Zusammensetzung, die zur Hemmung der Enkephalinase-Wirkung von Nutzen ist.

13. Verwendung einer nach ingendeinem der Ansprüche 1 bis 9 hergestellten Verbindung zur Herstellung einer pharmazeutischen Zusammensetzung für die orale Verabreichung zur Hemmung der Enkephalinase-Wirkung.

**Revendications pour les Etats contractants: BE CH DE FR GB IT LI LU NL SE**

1. Composé ayant la formule I

$$R_1C^*(HCOR_2){-}NH{-}C^*(HR_3){-}CONH(CH_2)_p{-}C^*(R_4R_5){-}COR_6$$

ou un mélange d'énantiomères ou diastéréoisomères le contenant, et leurs sels acceptables en pharmacie, où:

$R_1$ est alkyle ayant 1 à 6 atomes de carbone, adamantylméthyle, cycloalkylméthyle ayant 4 à 8 atomes de carbone ou $A{-}X_m{-}C_nH_{2n}{-}$ où X est oxygène ou soufre, A est phényle qui peut être substitué par le groupe Y, où Y est halogène, hydroxy, trifluorométhyle, alcoxy ayant 1 à 6 atomes de carbone, alkyle ayant 1 à 6 atomes de carbone, 2- ou 3-furanyle, 2- ou 3-thiényle ou phényle [qui peut être substitué par halogène, hydroxy, trifluorométhyle, alcoxy ayant 1 à 6 atomes de carbone ou alkyle ayant 1 à 6 atomes de carbone ou peut également être benzyle [dont le noyau phényle peut être substitué par le groupe Y, tel que défini ci-dessus], 1- ou 2-naphtyle, 2- ou 3-furanyle ou 2- ou 3-thiényle; m est 0 ou 1 et n est 0, 1, 2, 3 ou 4;

$R_2$ et $R_6$ peuvent être identiques ou différents et sont hydroxy, alcoxy ayant 1 à 8 atomes de carbone, $B{-}X_m{-}C_nH_{2n}{-}O{-}$ où B est phényle [qui peut être substitué par le groupe Y, tel que défini ici] ou 1- ou 2-naphtyle, X, m, et n sont tels que définis ici à condition que lorsque n = 0, m = 0, $-OCH_2OCO$-alkyle où le groupe alkyle a 1 à 6 atomes de carbone, $-OCH_2CO$-phényle [dont le noyau phényle peut être substitué par le groupe Y tel que défini ci-dessus], 1-glycéryle,

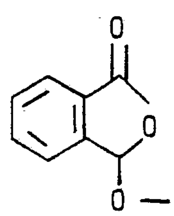

ou

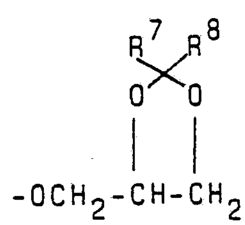

où $R_7$ est hydrogène, alkyle ayant 1 à 6 atomes de carbone ou phényle qui peut être substitué par le groupe Y, tel que défini ci-dessus, et $R_8$ est hydrogène ou alkyle ayant 1 à 6 atomes de carbone;

$R_2$ peut également être $-NR_7R_8$ où $R_7$ et $R_8$ sont tels que définis ci-dessus;

$R_3$ est alkyle ayant 1 à 6 atomes de carbone, cycloalkylméthyle ayant 4 à 8 atomes de carbone, 2- ou 3-thiénylméthyle, 2- ou 3-furanylméthyle, 1- ou 2-naphtylméthyle ou benzyle, dont le noyau phényle peut être substitué par le groupe Y tel que défini ci-dessus;

$R_4$ est $D{-}C_nH_{2n}{-}O_m{-}$ où D est hydrogène, alkyle ayant 1 à 4 atomes de carbone ou phényle qui peut être substitué par le groupe Z, où Z est halogène, hydroxy, trifluorométhyle, alcoxy ayant 1 à 6 atomes de carbone ou alkyle ayant 1 à 6 atomes de carbone;

m et n sont tels que définis ci-dessus;

$R_5$ esy hydrogène ou alkyle ayant 1 à 6 atomes de carbone; et

p est 0, 1 ou 2;
aux conditions qui suivent

(i) que, quand $R_4$ et $R_5$ sont tous deux de l'hydrogène et p est 1 ou 2, alors;

a) $R_1$ soit choisi parmi adamantyle, 4-phénylphényléthyle, $A—X_m—C_nH_{2n}—$ où m est 1, X est tel que défini ci-dessus et n est 1, 2, 3 ou 4, 1- ou 2-naphtyle, 2- ou 3-furanyle et 2- ou 3-thiényle; et/ou

b) $R_2$ et/ou $R_6$ soient choisis parmi $B—Xm—C_nH_{2n}—O—$ où B est tel que défini ci-dessus, m est un, X est tel que défini ci-dessus et n est 1, 2, 3 ou 4, $—OCH_2OCO$-alkyle où le groupe alkyle a 1 à 6 atomes de carbone, $—OCH_2CO$-phényle (où le groupe phényle peut être substitué par le groupe Y, tel que défini ici), 1-glycéryle,

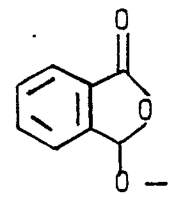 ou 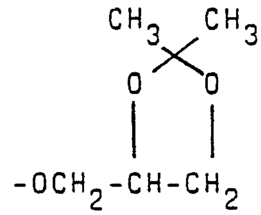

et $R_2$ peut également être $—NR_7R_8$ où $R_7$ et $R_8$ sont tels que définis ici; et/ou

c) $R_3$ soit 2- ou 3-furanylméthyle ou 4-phénylphényléthyle;

(ii) que, quand P est zéro, alors

$R_1$ soit benzyle ou benzylthiométhyle;

$R_3$ soit benzyle ou puisse être leucyle lorsque $R_1$ est benzyle;

$R_4$ soit benzyle quand $R_1$ est benzyle et soit méthyle quand $R_1$ est benzylthiométhyle;

$R_5$ soit hydrogène.

2. Composé de la formule I tel que défini à la revendication 1, caractérisé en ce que $R_4$ représente un groupe $D—C_nH_{2n}—O_m—$ tel que défini à la revendication 1 où la somme de n et m est au moins un.

3. Composé de la formule I tel que défini à la revendication 1 ou 2, caractérisé en ce que l'un de $R_2$ et $R_6$ est 2-phénoxyéthoxy, 1 glycéryle,

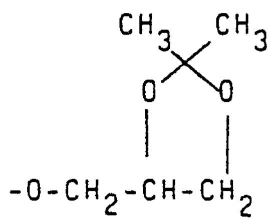 , 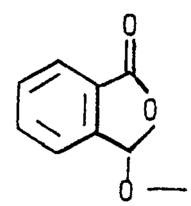 ,

ou (2,2-diméthyl-1-oxopropoxy)méthoxy, l'autre étant choisi dans les groupes définis ci-dessus ou est hydroxy, méthoxy, éthoxy ou benzyloxy.

4. Composé tel que défini à la revendication 2, caractérisé en ce que

$R_1$ est benzyle, facultativement *para* substitué par chlore, méthoxy, méthyle ou phényle, 2-phényléthyle ou 1- ou 2-naphtylméthyle,

$R_2$ et $R_6$ sont identiques ou différents et sont hydroxy, méthoxy, éthoxy, benzyloxy, 2-phénoxyéthoxy, 1-glycéryle,

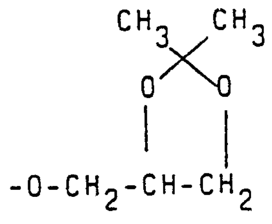 , 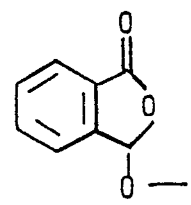 ,

ou (2,2-diméthyl-1-oxopropoxy)méthoxy, et

$R_3$ est benzyle, p-méthylbenzyle, p-phénylbenzyle, 1-naphtylméthyle ou 3-thiénylméthyle.

5. Composé selon la revendication 4, caractérisé en ce que $R_4$ est méthyle ou benzyle et $R_5$ est hydrogène.

6. Composé selon la revendication 3, caractérisé en ce que $R_1$ et $R_3$ sont tels que définis à la revendication 4, $R_4$ est hydrogène, méthyle ou benzyle et $R_5$ est hydrogène.

7. Composé selon la revendication 6, caractérisé en ce que $R_4$ est hydrogène.

8. Composé selon l'une quelconque des revendications précédentes où p est un.

9. N-[N-[L-[1-(2,2-diméthyl-1-oxopropoxy)méthoxy]carbonyl]-2-phényléthyl]-L-phénylalanyl]-β-alanine, ester (2,2-diméthyl-1-oxopropoxy)méthylique;

N-[N-[L-1-[phénylméthoxy]carbonyl]-2-phényléthyl]-L-phénylalanyl]-β-alanine, ester (2,2-diméthyl-1-oxopropoxy)méthylique;

N-[N-[L-1-carboxy-2-(4-phényl)phényléthyl]-L-phénylalanyl]-β-alanine;

N-[N-[L-[1-[(2-phénoxy)éthoxy)carbonyl]-2-(4-phényl)phényléthyl]-L-phénylalanyl]-β-alanine;

N-[N-[L-[1-[(1-oxo-3-isobenzofuranyloxy)]carbonyl]-2-(4-phényl)phényléthyl]-L-phénylalanyl]-β-alanine;

N-[N-[L-[1-[(2,3-dihydroxy)-1-propoxy]carbonyl]-2-(4-phényl)phényléthyl]-L-phénylalanyl]-β-alanine;

N-[N-[L-[1-[(2,2-diméthyl-1,3-dioxolan-4-yl)méthoxy]carbonyl]-2-(4-phényl)phényléthyl]-L-phénylalanyl]-β-alanine;

N-[N-[L-[1-[(2,2-diméthyl-1-oxopropoxy)méthoxy]carbonyl]-2-phényléthyl]-L-(4-phényl)phénylalanyl]-β-alanine;

N-[N-[L-[1-[(2-phénoxy)éthoxy]carbonyl]-2-phényléthyl]-L-(4 phényl)phénylalanyl]-β-alanine;

N-[N-[L-[1-[(1-oxo-3-isobenzofuranyloxy)carbonyl]-2-phényléthyl]-L-(4-phényl)phénylalanyl]-β-alanine;

N-[N-[L-[1-[(2,3-dihydroxy)-1-propoxy]carbonyl]-2-phényléthyl]-L-(4-phényl)phénylalanyl]-β-alanine;

N-[N-[L-[1-[(2,2-diméthyl-1,3-dioxolan-4-yl]méthoxy]carbonyl]-2-phényléthyl]-L-(4-phényl)phénylalanyl]-β-alanine;

N-[N-[L-[1-[(2,2-diméthyl-1-oxopropoxy)méthoxy]carbonyl]-2-(4-phényl)phényléthyl]-L-(4-phényl)phénylalanyl]-β-alanine;

N-[N-[L-[1-[(2-phénoxy)éthoxy]carbonyl]-2-(4-phényl)phényléthyl]-L-(4-phényl)phénylalanyl]-β-alanine;

N-[N-[L-[1-[(1-oxo-3-isobenzofuranyloxy)]carbonyl]-2-(4-phényl)phényléthyl]-L-(4-phényl)phénylalanyl]-β-alanine;

N-[N-[L-[1-[(2,3-dihydroxy)-1-propoxy]carbonyl]-2-(4-phényl)phényléthyl]-L-(4-phényl)phénylalanyl]-β-alanine;

N-[N-[L-[1-[(2,2-diméthyl-1,3-dioxolan-4-yl)méthoxy]carbonyl]-2-(4-phényl)phényléthyl]-L-(4-phényl)-phénylalanyl]-β-alanine

N-[N-[L-[1-[(2,2-diméthyl-1-oxopropoxy)méthoxy]carbonyl]-2-phényléthyl]-L-phénylalanyl]-D,L-α-méthyl-β-alanine;

N-[N-[L-[1-[(2-phénoxy)éthoxy]carbonyl]-2-phényléthyl]-L-phénylalanyl]-D,L-α-méthyl-β-alanine;

N-[N-[L-[1-[(1-oxo-3-isobenzofuranyloxy)]carbonyl]-2-phényléthyl]-L-phénylalanyl]-D-,L-α-méthyl-β-alanine;

N-[N-[L-[1-[(2,3-dihydroxy)-1-propoxy]carbonyl]-2-phényléthyl]-L-phénylalanyl]-D,L-α-méthyl-β-alanine;

N-[N-[L-[1-[(2,2-diméthyl-1,3-dioxolan-4-yl)méthoxy]carbonyl]-2-phényléthyl]-L-phénylalanyl]-D,L-α-méthyl-β-alanine;

N-[N-[L-[1-[(2,2-diméthyl-1-oxopropoxy)méthoxy]carbonyl]-2-(4-phényl)phényléthyl]-L-phénylalanyl]-D,L-α-méthyl-β-alanine;

N-[N-[L-[1-[(2-phénoxy)éthoxy]carbonyl]-2-(4-phényl)phényléthyl]-L-phénylalanyl]-D,L-α-méthyl-β-alanine;

N-[N-[L-[1-[(1-oxo-3-isobenzofuranyloxy)]carbonyl]-2-(4-phényl)phényléthyl]-L-phénylalanyl]-D,L-α-méthyl-β-alanine;

N-[N-[L-[1-[(2,3-dihydroxy)-1-propoxy]carbonyl]-2-(4-phényl)phényléthyl]-L-phénylalanyl]-D,L-α-méthyl-β-alanine;

N-[N-[L-[1-[(2,2-diméthyl-1,3-dioxolan-4-yl)méthoxy]carbonyl]-2-(4-phényl)phényléthyl]-L-phénylalanyl]-D,L-α-méthyl-β-alanine;

N-[N-[L-[1-[(2,2-diméthyl-1-oxopropoxy)méthoxy]carbonyl]-2-phényléthyl]-L-(4-phényl)phénylalanyl]-D,L-α-méthyl-β-alanine;

N-[N-[L-[1-[(2-phénoxy)éthoxy]carbonyl]-2-phényléthyl]-L-(4-phényl)phénylalanyl]-D,L-α-méthyl-β-alanine;

N-[N-[L-[1-[(1-oxo-3-isobenzofuranyloxy)]carbonyl]-2-phényléthyl]-L-(4-phényl)phénylalanyl]-D,L-α-méthyl-β-alanine;

N-[N-[L-[1-[(2,3-dihydroxy)-1-propoxy]carbonyl]-2-phényléthyl]-L-(4-phényl)phénylalanyl]-D,L-α-méthyl-β-alanine;

N-[N-[L-[1-[(2,2-diméthyl-1,3-dioxolan-4-yl)méthoxy]carbonyl]-2-phényléthyl]-L-(4-phényl)phénylalanyl]-D,L-α-méthyl-β-alanine;

N-[N-[L-[1-[(2,2-diméthyl-1-oxopropoxy)méthoxy]carbonyl]-2-(4-phényl)phényléthyl]-L-(4-phényl)-phénylalanyl]-D,L-α-méthyl-β-alanine;

N-[N-[L-[1-[(2-phénoxy)éthoxy]carbonyl]-2-(4-phényl)-phényléthyl]-L-(4-phényl)phénylalanyl]-D,L-α-méthyl-β-alanine;

N-[N-[L-[1-[(1-oxo-3-isobenzofuranyloxy)]carbonyl]-2-(4-phényl)phényléthyl]-L-(4-phényl)phénylalanyl]-D,L-α-méthyl-β-alanine;

N-[N-[L-[1-[(2,3-dihydroxy)-1-propoxy]carbonyl]-2-(4-phényl)phényléthyl]-L-(4-phényl)phénylalanyl]-D,L-α-méthyl-β-alanine;

N-[N-[L-[1-[(2,2-diméthyl-1,3-dioxolan-4-yl)méthoxy]carbonyl]-2-(4-phényl)phényléthyl]-L-(4-phényl)-

phénylalanyl]-D,L-α-méthyl-β-alanine;

N-[N-[L-[1-[(2,2-diméthyl-1-oxopropoxy)méthoxy]carbonyl]-2-phényléthyl]-L-2-thiénylalanyl]-β-alanine;

N-[N-[L-[1-[(2,2-diméthyl-1-oxopropoxy)méthoxy]carbonyl]-2-phényléthyl]-L-3-thiénylalanyl]-β-alanine;

N-[N-[L-[1-[(2,2-diméthyl-1-oxopropoxy)méthoxy]carbonyl]-2-phényléthyl]-L-2-furoalanyl]-β-alanine;

N-[N-[L-[1-[(2,2-diméthyl-1-oxopropoxy)méthoxy]carbonyl]-2-phényléthyl]-L-phénylalanyl]-α-méthylalanine;

N-[N-[L-[1-[(2,2-diméthyl-1-oxopropoxy)méthoxy]carbonyl]-2-phényléthyl]-L-phénylalanyl]-L-α-hydroxy-β-alanine;

N-[N-[L-[1-[(2,2-diméthyl-1-oxopropoxy)méthoxy]carbonyl]-2-phényléthyl]-L-phénylalanyl]-D-α-hydroxy-β-alanine;

N-[N-[L-[1-[(2,2-diméthyl-1-oxopropoxy)méthoxy]carbonyl]-2-phényléthyl]-L-phénylalanyl]-L-α-méthoxy-β-alanine;

N-[N-[L-[1-[(2,2-diméthyl-1-oxopropoxy)méthoxy]carbonyl]-2-phényléthyl]-L-phénylalanyl]-D-α-méthoxy-β-alanine;

Ester benzylique de N-[N-[L-[1-[(2,2-diméthyl-1-oxopropoxy)méthoxy]carbonyl]-2-phényléthyl]-L-phénylalanyl]-D,L-α-méthyl-β-alanine;

N-[N-[L-[1-(2,2-diméthyl-1-oxopropoxy)méthoxy]carbonyl]-2-(4-méthoxy)phényléthyl]-L-phénylalanyl-β-alanine;

N-[(L)-1-carboxy-2 phényléthyl]-L-phényl alanyl-L-phénylalanine;

N-[(L)-1-carboxy-2-phényléthyl]-L-leucyl-L-phénylalanine; et/ou

un sel de l'un de ceux qui précèdent, qui contient une fonction d'acide libre, avec une base acceptable en pharmacie ou un sel de l'un de ceux qui précèdent avec un acide acceptable en pharmacie.

10. Composition qui comprend un composé tel que défini selon l'une quelconque des revendications précédentes en combinaison avec un véhicule acceptable en pharmacie.

11. Utilisation d'un composé selon l'une quelconque des revendications 1 à 9 pour la préparation d'une composition pharmaceutique utile pour inhiber l'action de l'encéphalinase.

12. Utilisation d'un composé selon l'une quelconque des revendications 1 à 9 pour la préparation d'une composition pharmaceutique pour une administration orale pour inhiber l'action de l'encéphalinase.

**Revendications pour l'Etat contractant: AT**

1. Méthode de préparation d'un composé ayant la formule I

$$R_1C^*(HCOR_2)—NH—C^*(HR_3)—CONH(CH_2)_p—C^*(R_4R_5)—COR_6$$

ou un mélange d'énantiomères ou diastéréoisomères le contenant, et leurs sels acceptables en pharmacie, où:

$R_1$ est alkyle ayant 1 à 6 atomes de carbone, adamantylméthyle, cycloalkylméthyle ayant 4 à 8 atomes de carbone ou $A—X_m—C_nH_{2n}—$ où X est oxygène ou soufre, A est phényle qui peut être substitué par le groupe Y, où Y est halogène, hydroxy, trifluorométhyle, alcoxy ayant 1 à 6 atomes de carbone, alkyle ayant 1 à 6 atomes de carbone, 2- ou 3-furanyle, 2- ou 3-thiényle ou phényle [qui peut être substitué par halogène, hydroxy, trifluorométhyle, alcoxy ayant 1 à 6 atomes de carbone ou alkyle ayant 1 à 6 atomes de carbone] ou peut également être benzyle [dont le noyau phényle peut être substitué par le groupe Y, tel que défini ci-dessus], 1- ou 2-naphtyle, 2- ou 3-furanyle ou 2- ou 3-thiényle; m est 0 ou 1 et n est 0, 1, 2, 3 ou 4;

$R_2$ et $R_6$ peuvent être identiques ou différents et sont hydroxy, alcoxy ayant 1 à 8 atomes de carbone, $B—X_m—C_nH_{2n}—O—$ où B est phényle [qui peut être substitué par le groupe Y, tel que défini ici] ou 1- ou 2-naphtyle, X, m, et n sont tels que définis ici à condition que lorsque n = 0, m = 0, $—OCH_2OCO$-alkyle où le groupe alkyle a 1 à 6 atomes de carbone, $—OCH_2CO$-phényle [dont le noyau phényle peut être substitué par le groupe Y tel que défini ci-dessus], 1-glycéryle,

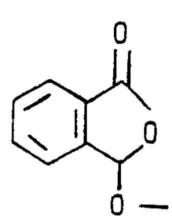

ou

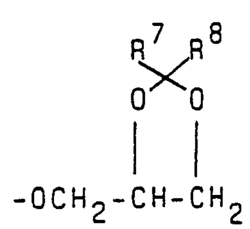

où $R_7$ est hydrogène, alkyle ayant 1 à 6 atomes de carbone ou phényle qui peut être substitué par le groupe Y, tel que défini ci-dessus, et $R_8$ est hydrogène ou alkyle ayant 1 à 6 atomes de carbone;

$R_2$ peut également être $—NR_7R_8$ où $R_7$ et $R_8$ sont tels que définis ci-dessus;

$R_3$ est alkyle ayant 1 à 6 atomes de carbone, cycloalkylméthyle ayant 4 à 8 atomes de carbone, 2- ou 3-thiénylméthyle, 2- ou 3-furanylméthyle, 1- ou 2-naphtylméthyle ou benzyle, dont le noyau phényle peut être substitué par le groupe Y tel que défini ci-dessus;

$R_4$ est $D—C_nH_{2n}—O_m—$ où D est hydrogène, alkyle ayant 1 à 4 atomes de carbone ou phényle qui peut être substitué par le groupe Z, où Z est halogène, hydroxy, trifluorométhyle, alcoxy ayant 1 à 6 atomes de carbone ou alkyle ayant 1 à 6 atomes de carbone;

m et n sont tels que définis ci-dessus;

$R_5$ esy hydrogène ou alkyle ayant 1 à 6 atomes de carbone; et

p est 0, 1 ou 2;

aux conditions qui suivent

(i) que, quand $R_4$ et $R_5$ sont tous deux de l'hydrogène et p est 1 ou 2, alors;

a) $R_1$ soit choisi parmi adamantyle, 4-phénylphényléthyle, $A—X_m—C_nH_{2n}—$ où m est 1, X est tel que défini ci-dessus et n est 1, 2, 3 ou 4, 1- ou 2-naphtyle, 2- ou 3-furanyle et 2- ou 3-thiényle; et/ou

b) $R_2$ et/ou $R_6$ soient choisis parmi $B—Xm—C_nH_{2n}—O—$ où B est tel que défini ci-dessus, m est un, X est tel que défini ci-dessus et n est 1, 2, 3 ou 4, $—OCH_2OCO$-alkyle où le groupe alkyle a 1 à 6 atomes de carbone, $—OCH_2CO$-phényle (où le groupe phényle peut être substitué par le groupe Y, tel que défini ici), 1-glycéryle,

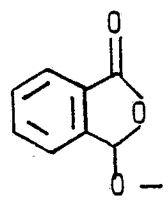

ou

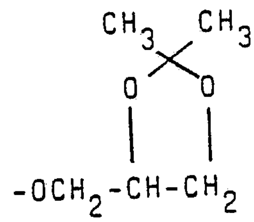

et $R_2$ peut également être $—NR_7R_8$ où $R_7$ et $R_8$ sont tels que définis ici; et/ou

c) $R_3$ soit 2- ou 3-furanylméthyle ou 4-phénylphényléthyle.

(ii) que, quand P est zéro, alors

$R_1$ soit benzyle ou benzylthiométhyle;

$R_3$ soit benzyle ou puisse être leucyle lorsque $R_1$ est benzyle;

$R_4$ soit benzyle quand $R_1$ est benzyle et soit méthyle quand $R_1$ est benzylthiométhyle;

$R_5$ soit hydrogène.

caractérisée en ce que le composé est préparé par une méthode appropriée choisie parmi les méthodes suivantes (où, dans les formules suivantes, p, $R_1$, $R_2$, $R_3$, $R_4$, $R_5$ et $R_6$ sont tels que définis ci-dessus, comprenant une protection appropriée de tout groupe réactif);

a) la réduction d'un composé de formule XX à la double liaison C=N

$$R_1—\underset{\underset{COR_2}{|}}{C}=N—\overset{\overset{R_3}{|}}{CH}—CONH(CH_2)p—CR_4R_5COR_6 \quad \rightarrow I;$$

XX

b) la condensation d'un cétoacide ou ester de formule II avec un aminoacide de formule III dans un milieu réactif contenant un agent réducteur

$$\underset{II}{R_1—C(O)COR_2} + \underset{III}{H_2N\overset{\overset{R_3}{|}}{C}HCONH(CH_2)_pCR_4R_5COR_6} \quad \rightarrow I;$$

c) le couplage d'un aminoacide de formule VIII avec un aminoacide de formule V

$$\underset{VIII.}{R_1—\underset{\underset{COR_2}{|}}{C}NH\overset{\overset{R_3}{|}}{C}H—CO_2H} + \underset{V}{H_2N(CH_2)pCR_4R_5COR_6} \quad \rightarrow I$$

d) la réduction d'un composé de formule XXI à la double liaison C=N

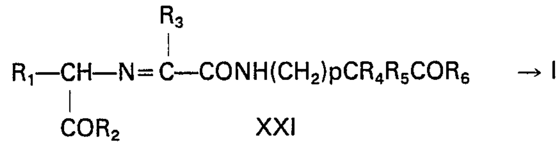

XXI

e) la condensation d'un cétoacide ou ester de formule X avec un aminoacide dans un milieu réactif contenant un agent réducteur

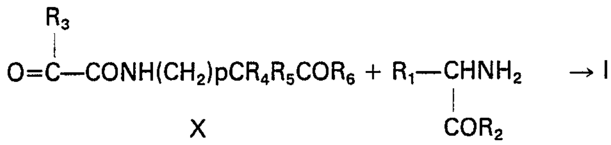

X

f) l'alkylation d'une amine de formule III avec un composé de formule XXII

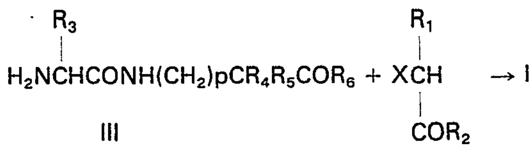

III

XXII

où X est un atome d'halogène;
avec ensuite enlèvement de tous les groupes protecteurs si nécessaire pour obtenir le composé souhaité de formule I et ensuite, si on le souhaite, la conversion du composé de formule I en un autre composé de formule I et/ou la formation de son sel, et si on le souhaite l'isolement d'un isomère préféré.

2. Méthode telle que définie à la revendication 1, caractérisée en ce qu'un composé de formule I est produit où $R_4$ représente le groupe $D—C_nH_{2n}—O_m—$ tel que défini à la revendication 1 où la somme de n et m est d'au moins un.

3. Méthode selon la revendication 1 ou 2, caractérisée en ce qu'un composé de formule I est produit où l'un de $R_2$ et $R_6$ est 2-phénoxyéthoxy, 1-glycéryle,

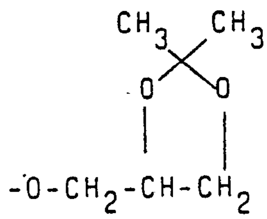 , 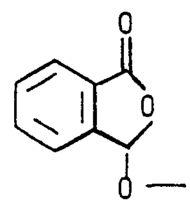 ,

ou (2,2-diméthyl-1-oxopropoxy)méthoxy et l'autre est choisi parmi les groupes définis ci-dessus ou est hydroxy, méthoxy, éthoxy ou benzyloxy.

4. Méthode telle que définie à la revendication 1 ou 2, caractérisée en ce qu'un composé est produit où

$R_1$ est benzyle facultativement *para* substitué par chlore, méthoxy, méthyle ou phényle, 2-phényléthyle ou 1- ou 2-naphtylméthyle,

$R_2$ et $R_6$ sont identiques ou différents et sont hydroxy, méthoxy, éthoxy, benzyloxy, 2-phénoxyéthoxy, 1-glycéryle,

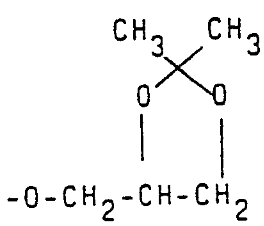 , 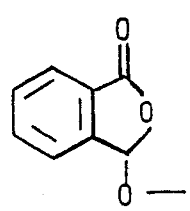 ,

ou (2,2-diméthyl-1-oxopropoxy)méthoxy, et

$R_3$ est benzyle, p-méthylbenzyle, p-phénylbenzyle, 1-naphtylméthyle ou 3-thiénylméthyle.

5. Méthode telle que définie à la revendication 4, caractérisée en ce qu'un composé est produit dans lequel $R_4$ est méthyle ou benzyle et $R_5$ est hydrogène.

6. Méthode telle que définie à la revendication 3, caractérisée en ce qu'un composé est produit où $R_1$ et

$R_3$ sont tels que définis à la revendication 4, $R_4$ est hydrogène, méthyle ou benzyle et $R_5$ est hydrogène.

7. Méthode telle que définie à la revendication 6, caractérisée en ce que $R_4$ est hydrogène.

8. Méthode telle que définie selon l'une quelconque des revendications précédentes, caractérisée en ce qu'un composé est produit où p est un.

9. Méthode telle que définie à la revendication 1, caractérisée en ce que le composé

N-[N-[L-[1-(2,2-diméthyl-1-oxopropoxy)méthoxy]carbonyl]-2-phényléthyl]-L-phénylalanyl]-β-alanine, ester (2,2-diméthyl-1-oxopropoxy)méthylique;

N-[N-[L-[1-[phénoxyméthoxy]carbonyl]-2-phényléthyl]-L-phénylalanyl]-β-alanine, ester (2,2-diméthyl-1-oxopropoxy)méthylique;

N-[N-[L-1-carboxy-2-(4-phényl)phényléthyl]-L-phénylalanyl]-β-alanine;

N-[N-[L-[1-[(2,2-diméthyl-1-oxopropoxy)méthoxy]carbonyl]-2-(4-phényl)phényléthyl]-L-phénylalanyl]-β-alanine, ester (2,2-diméthyl-1-oxopropoxy)méthylique;

N-[N-[L-1-carboxy-2-phényléthyl]-L-(4-phényl)phénylalanyl]-β-alanine;

N-[N-[L-[1-[(2,2-diméthyl-1-oxopropoxy)méthoxy]carbonyl]-2-(4-phényl)phényléthyl]-L-(4-phényl)-phénylalanyl-β-alanine, ester(2,2-diméthyl-1-oxopropoxy)méthylique;

N-[N-[L-[1-[(2,2-diméthyl-1-oxopropoxy)méthoxy]carbonyl]-2-phényléthyl]-L-(4-phényl)phénylalanyl]-β-alanine, ester(2,2-diméthyl-1-oxopropoxy)méthylique;

N-[N-[L-1-carboxy-2-phényléthyl]-L-phénylalanyl]-L-(α-méthyl)-β-alanine;

N-[N-[L-[1-[(2,2-diméthyl-1-oxopropoxy)méthoxy]carbonyl]-2-phényléthyl]-L-phénylalanyl]-L-(α-méthyl)-β-alanine, ester(2,2-diméthyl-1-oxopropoxy)méthylique;

N-[N-[(L-1-carboxy-2-phényléthyl)]-L-phénylalanyl]-β-alanine, ester 2-phénoxyéthylique;

N-[N-[L-[1-[(2,2-diméthyl-1-oxopropoxy)méthoxy]carbonyl]-2-phényléthyl]-L-phénylalanyl]-β-alanine;

N-[N-[L-[1-[(2-phénoxy)éthoxy]carbonyl]-2-phényléthyl]-L-phénylalanyl]-β-alanine;

N-[N-[L-[1-[(1-oxo-3-isobenzofuranyloxy)]carbonyl]-2-phényléthyl]-L-phénylalanyl]-β-alanine;

N-[N-[L-[1-[(2,3-dihydroxy)-1-propoxy]carbonyl]-2-phényléthyl]-L-phénylalanyl]-β-alanine;

N-[N-[L-[1-[(2,2-diméthyl-1,3-dioxolan-4-yl)méthoxy]carbonyl]-2-phényléthyl]-L-phénylalanyl]-β-alanine;

N-[N-[L-[1-[(2,2-diméthyl-1-oxopropoxy)méthoxy]carbonyl]-2-(4-phényl)phényléthyl]-L-phénylalanyl]-β-alanine;

N-[N-[L-[1-[(2-phénoxy)éthoxy)carbonyl]-2-(4-phényl)phényléthyl]-L-phénylalanyl]-β-alanine;

N-[N-[L-[1-[(1-oxo-3-isobenzofuranyloxy)]carbonyl]-2-(4-phényl)phényléthyl]-L-phénylalanyl]-β-alanine;

N-[N-[L-[1-[(2,3-dihydroxy)-1-propoxy]carbonyl]-2-(4-phényl)phényléthyl]-L-phénylalanyl]-β-alanine;

N-[N-[L-[1-[(2,2-diméthyl-1,3-dioxolan-4-yl)méthoxy]carbonyl]-2-(4-phényl)phényléthyl]-L-phénylalanyl]-β-alanine;

N-[N-[L-[1-[(2,2-diméthyl-1-oxopropoxy)méthoxy]carbonyl]-2-phényléthyl]-L-(4-phényl)phénylalanyl]-β-alanine;

N-[N-[L-[1-[(2-phénoxy)éthoxy]carbonyl]-2-phényléthyl]-L-(4 phényl)phénylalanyl]-β-alanine;

N-[N-[L-[1-[(1-oxo-3-isobenzofuranyloxy)carbonyl]-2-phényléthyl]-L-(4-phényl)phénylalanyl]-β-alanine;

N-[N-[L-[1-[(2,3-dihydroxy)-1-propoxy]carbonyl]-2-phényléthyl]-L-(4-phényl)phénylalanyl]-β-alanine;

N-[N-[L-[1-[(2,2-diméthyl-1,3-dioxolan-4-yl]méthoxy]carbonyl]-2-phényléthyl]-L-(4-phényl)phénylalanyl]-β-alanine;

N-[N-[L-[1-[(2,2-diméthyl-1-oxopropoxy)méthoxy]carbonyl]-2-(4-phényl)phényléthyl]-L-(4-phényl)-phénylalanyl]-β-alanine;

N-[N-[L-[1-[(2-phénoxy)éthoxy]carbonyl]-2-(4-phényl)phényléthyl]-L-(4-phényl)phénylalanyl]-β-alanine;

N-[N-[L-[1-[(1-oxo-3-isobenzofuranyloxy)]carbonyl]-2-(4-phényl)phényléthyl]-L-(4-phényl)phénylalanyl]-β-alanine;

N-[N-[L-[1-[(2,3-dihydroxy)-1-propoxy]carbonyl]-2-(4-phényl)phényléthyl]-L-(4-phényl)phénylalanyl]-β-alanine;

N-[N-[L-[1-[(2,2-diméthyl-1,3-dioxolan-4-yl)méthoxy]carbonyl]-2-(4-phényl)phényléthyl]-L-(4-phényl)-phénylalanyl]-β-alanine

N-[N-[L-[1-[(2,2-diméthyl-1-oxopropoxy)méthoxy]carbonyl]-2-phényléthyl]-L-phénylalanyl]-D,L-α-méthyl-β-alanine;

N-[N-[L-[1-[(2-phénoxy)éthoxy]carbonyl]-2-phényléthyl]-L-phénylalanyl]-D,L-α-méthyl-β-alanine;

N-[N-[L-[1-[(1-oxo-3-isobenzofuranyloxy)]carbonyl]-2-phényléthyl]-L-phénylalanyl]-D-,L-α-méthyl-β-alanine;

N-[N-[L-[1-[(2,3-dihydroxy)-1-propoxy]carbonyl]-2-phényléthyl]-L-phénylalanyl]-D,L-α-méthyl-β-alanine;

N-[N-[L-[1-[(2,2-diméthyl-1,3-dioxolan-4-yl)méthoxy]carbonyl]-2-phényléthyl]-L-phénylalanyl]-D,L-α-méthyl-β-alanine;

N-[N-[L-[1-[(2,2-diméthyl-1-oxopropoxy)méthoxy]carbonyl]-2-(4-phényl)phényléthyl]-L-phénylalanyl]-D,L-α-méthyl-β-alanine;

N-[N-[L-[1-[(2-phénoxy)éthoxy]carbonyl]-2-(4-phényl)phényléthyl]-L-phénylalanyl]-D,L-α-méthyl-β-alanine;

N-[N-[L-[1-[(1-oxo-3-isobenzofuranyloxy)]carbonyl]-2-(4-phényl)phényléthyl]-L-phénylalanyl]-D,L-α-méthyl-β-alanine;

N-[N-[L-[1-[(2,3-dihydroxy)-1-propoxy]carbonyl]-2-(4-phényl)phényléthyl]-L-phénylalanyl]-D,L-α-méthyl-β-alanine;

N-[N-[L-[1-[(2,2-diméthyl-1,3-dioxolan-4-yl)méthoxy]carbonyl]-2-(4-phényl)phényléthyl]-L-phénylalanyl]-D,L-α-méthyl-β-alanine;

N-[N-[L-[1-[(2,2-diméthyl-1-oxopropoxy)méthoxy]carbonyl]-2-phényléthyl]-L-(4-phényl)phénylalanyl]-D,L-α-méthyl-β-alanine;

N-[N-[L-[1-[(2-phénoxy)éthoxy]carbonyl]-2-phényléthyl]-L-(4-phényl)phénylalanyl]-D,L-α-méthyl-β-alanine;

N-[N-[L-[1-[(1-oxo-3-isobenzofuranyloxy)]carbonyl]-2-phényléthyl]-L-(4-phényl)phénylalanyl]-D,L-α-méthyl-β-alanine;

N-[N-[L-[1-[(2,3-dihydroxy)-1-propoxy]carbonyl]-2-phényléthyl]-L-(4-phényl)phénylalanyl]-D,L-α-méthyl-β-alanine;

N-[N-[L-[1-[(2,2-diméthyl-1,3-dioxolan-4-yl)méthoxy]carbonyl]-2-phényléthyl]-L-(4-phényl)phénylalanyl]-D,L-α-méthyl-β-alanine;

N-[N-[L-[1-[(2,2-diméthyl-1-oxopropoxy)méthoxy]carbonyl]-2-(4-phényl)phényléthyl]-L-(4-phényl)-phénylalanyl]-D,L-α-méthyl-β-alanine;

N-[N-[L-[1-[(2-phénoxy)éthoxy]carbonyl]-2-(4-phényl)-phényléthyl]-L-(4-phényl)phénylalanyl]-D,L-α-méthyl-β-alanine;

N-[N-[L-[1-[(1-oxo-3-isobenzofuranyloxy)]carbonyl]-2-(4-phényl)phényléthyl]-L-(4-phényl)phénylalanyl]-D,L-α-méthyl-β-alanine;

N-[N-[L-[1-[(2,3-dihydroxy)-1-propoxy]carbonyl]-2-(4-phényl)phényléthyl]-L-(4-phényl)phénylalanyl]-D,L-α-méthyl-β-alanine;

N-[N-[L-[1-[(2,2-diméthyl-1,3-dioxolan-4-yl)méthoxy]carbonyl]-2-(4-phényl)phényléthyl]-L-(4-phényl)-phénylalanyl]-D,L-α-méthyl-β-alanine;

N-[N-[L-[1-[(2,2-diméthyl-1-oxopropoxy)méthoxy]carbonyl]-2-phényléthyl]-L-2-thiénylalanyl]-β-alanine;

N-[N-[L-[1-[(2,2-diméthyl-1-oxopropoxy)méthoxy]carbonyl]-2-phényléthyl]-L-3-thiénylalanyl]-β-alanine;

N-[N-[L-[1-[(2,2-diméthyl-1-oxopropoxy)méthoxy]carbonyl]-2-phényléthyl]-L-2-furoalanyl]-β-alanine;

N-[N-[L-[1-[(2,2-diméthyl-1-oxopropoxy)méthoxy]carbonyl]-2-phényléthyl]-L-phénylalanyl]-α-méthylalanine;

N-[N-[L-[1-[(2,2-diméthyl-1-oxopropoxy)méthoxy]carbonyl]-2-phényléthyl]-L-phénylalanyl]-L-α-hydroxy-β-alanine;

N-[N-[L-[1-[(2,2-diméthyl-1-oxopropoxy)méthoxy]carbonyl]-2-phényléthyl]-L-phénylalanyl]-D-α-hydroxy-β-alanine;

N-[N-[L-[1-[(2,2-diméthyl-1-oxopropoxy)méthoxy]carbonyl]-2-phényléthyl]-L-phénylalanyl]-L-α-méthoxy-β-alanine;

N-[N-[L-[1-[(2,2-diméthyl-1-oxopropoxy)méthoxy]carbonyl]-2-phényléthyl]-L-phénylalanyl]-D-α-méthoxy-β-alanine;

Ester benzylique de N-[N-[L-[1-[(2,2-diméthyl-1-oxopropoxy)méthoxy]carbonyl]-2-phényléthyl]-L-phénylalanyl]-D,L-α-méthyl-β-alanine;

N-[N-[L-[1-(2,2-diméthyl-1-oxopropoxy)méthoxy]carbonyl]-2-(4-méthoxy)phényléthyl]-L-phénylalanyl-β-alanine;

N-[(L)-1-carboxy-2 phényléthyl]-L-phényl alanyl-L-phénylalanine;

N-[(L)-1-carboxy-2-phényléthyl]-L-leucyl-L-phénylalanine; et/ou

ou un sel acceptable en pharmacie est produit.

10. Méthode de fabrication d'une composition pharmaceutique, caractérisée en ce qu'un composé de formule I tel que défini à la revendication 1 ou un mélange d'énantiomères ou diastéréoisomères le contenant ou son sel acceptable en pharmacie, est mélangé à un véhicule ou excipient acceptable en pharmacie.

11. Méthode de fabrication d'une composition pharmaceutique, caractérisée en ce qu'un composé de formule I tel que défini à la revendication 1 ou un mélange d'énantiomères ou diastéréoisomères le contenant ou bien son sel acceptable en pharmacie, préparé par le procédé selon l'une quelconque des revendications précédentes, est mélangé à un véhicule ou excipient acceptable en pharmacie.

12. Utilisation d'un composé produit selon l'une quelconque des revendications 1 à 9 pour la préparation d'une composition pharmaceutique utile pour inhiber l'action de l'encéphalinase.

13. Utilisation d'un composé produit selon l'une quelconque des revendications 1 à 9 pour préparer une composition pharmaceutique pour administration orale pour inhiber l'action de l'encéphalinase.